# EUROPEAN PATENT SPECIFICATION

(11) **EP 3 851 455 B1**
(45) Date of publication and mention of the grant of the patent: **12.07.2023**
(21) Application number: 18934071.4
(22) Date of filing: 27.09.2018
(51) Int. Cl.: C07K 16/28, C12N 15/13, C12N 15/70, C07K 1/22, A61K 39/395, A61P 35/00

(54) **MONOCLONAL ANTIBODY SPECIFICALLY BINDING HUMAN AND MONKEY CD38 ANTIGENS, PREPARATION METHOD AND USE THEREOF**
MONOKLONALER ANTIKÖRPER, SPEZIFISCH BINDEN FÜR DAS CD38-ANTIGEN VON MENSCH UND AFFE, METHODEN ZU DESSEN HERSTELLUNG UND VERWENDUNG
ANTICORPS MONOCLONAL SE LIANT DE MANIÈRE SPÉCIFIQUE À L'ANTIGÈNE CD38 HUMAIN ET DE SINGE, SON PROCÉDÉ DE PRÉPARATION ET SON UTILISATION

(30) Priority: 19.09.2018 CN 201811090932
(43) Date of publication of application: 21.07.2021
(73) Proprietor: Acrolmmune BioPharmaceutical Co., Ltd., Nanjing (Hi-Tech Park) (CN)
(72) Inventor: HU, Hongqun, Suzhou, Jiangsu 215123 (CN); SONG, Xiaoqi, Suzhou, Jiangsu 215123 (CN); CHEN, Zui, Suzhou, Jiangsu 215123 (CN); MA, Xiaoxiao, Suzhou, Jiangsu 215123 (CN); YUAN, Yanping, Suzhou, Jiangsu 215123 (CN); ZHOU, Qunmin, Suzhou, Jiangsu 215123 (CN)
(74) Representative: Petculescu, Ana-Maria
(86) International application number: PCT/CN2018/107812
(87) International publication number: WO 2020/056790

(56) References cited:
- WO-A1-2006/099875
- WO-A1-2018/013917
- WO-A1-2018/013917
- WO-A1-2018/083204
- WO-A2-2016/022589
- CN-A- 107 033 243
- CN-A- 107 365 385
- JP-A- 2016 034 954
- US-A1- 2018 117 150
- GALSON JACOB D. ET AL: "Studying the antibody repertoire after vaccination: practical applications", TRENDS IN IMMUNOLOGY, vol. 35, no. 7, 1 July 2014 (2014-07-01), pages 319-331, XP055918888, GB ISSN: 1471-4906, DOI: 10.1016/j.it.2014.04.005

## Description

### TECHINCAL FIELD OF THE INVENTION

The present invention belongs to the field of biotechnology involving monoclonal antibodies. The present invention relates to a monoclonal antibody, specifically binds to human and monkey CD38 antigen, as well as preparation method and use thereof.

### BACKGROUND

CD38 (also known as T10, an antigen recognized by OKT10 monoclonal antibody) is a 45KD type II transmembrane glycoprotein. The cDNAs encoding human CD38 and mouse CD38 gene were cloned and reported in the early 1990s (Jackson DG and Bell JI. J. Immunol. 1990, 144:2811-2815; Harada et al. J Immunol. 1993, 151:3111-8); The cDNA encoding cynomolgus macaque CD38 gene was cloned and reported in 2004 (Ferrero E et al. BMC Immunol. 2004, 5:21). There are 300 amino acids in the full length human CD38 protein. Among them, 21 amino acids at the N-terminal are located inside the cell, 22 amino acids located in the cell membrane, and 257 amino acids at the C-terminal located outside the cell. There are 301 amino acids in the full length cynomolgus macaque CD38 protein, with a 92% identity to the amino acid sequence of human CD38 protein; while there are 304 amino acids in the full length mouse CD38 protein, with about 70% identity to the amino acid sequence of human CD38 protein.

The earliest report regarding the biological function of CD38 was derived from a paper published in 1992 by States DJ, et al. (States DJ, Walseth TF and Lee HC. Trends Biochem. Sci. 1992, 17: 495). In this report, States DJ, etc. noticed, for the first time, that CD38 molecule had sequence and structure identities to the Aplysia ADP-ribosyl cyclase and speculated that CD38 should have Aplysia ADP-ribosyl cyclase activity, and would catalyze the conversion of NAD⁺ into cADPR, in which cADPR serving as a second messenger within cells and mediating Ca²⁺ mobilization. Therefore, CD38 is also called ADP-ribosyl cyclase/cyclic ADP-ribose hydrolase 1 (ADPR1). Subsequent studies by others also confirmed this speculation and found that CD38 protein molecules also have dehydrogenase activity, which can further catalyze cADPR into ADPR (Howard M et al. Science. 1993; 262:105 ; Summerhill RJ, Jackson DG, Galione A. FEBS Lett. 1993,335:231-3; Prasad GS et al., Nature Structural Biology 19963:957-964). Therefore, CD38 has bi-functional enzyme activities, and the active sites of these bi-functional enzymes are located in the extracellular region. The overview of the CD38 molecular structure and its enzymatic activity and function thereof can be referred to review papers (Mehta K, Shahid U and Malavasi F: Human CD38, a cell-surface protein with multiple function. FASEB J. 1996, 10; 1408-1417; George Shubinsky, Michael Schlesinger: The CD38 lymphocyte differentiation marker: new insight into its ectoenzymatic activity and its role as a signal transducer. Immunity 1997, 7:315-324).

CD38 (T10) antigen was first discovered and reported by Feinnerz E and colleagues in 1980, and they observed that normal thymocytes and human T-lymphoma cell line MOLT-4 expressing an antigen can be specifically bound by a monoclonal antibody OKT10, and the antigen was then called as T10 at that time (Feinnerz E et al. PNAS 1980, 77:1588-1592). Other studies since then have demonstrated that CD38 (T10) antigen was widely expressed in other human cells such as B-lymphocytes, macrophages, dendritic cells, platelets, bone marrow plasma cells and other blood and lymphoid cells except T-lymphocytes. In addition, different levels of CD38 (T10) antigens were also expressed in other tissue cells such as nerve cells and glial cells in the central nervous system, peripheral nerve cells, islet cells in the pancreas, osteoclasts in the bone tissue, skeletal muscle cells, cardiomyocytes, and bronchial epithelial cells (See the following review: Mehta K, Shahid U and Malavasi F: Human CD38, a cell-surface protein with multiple function. FASEB J 10, 1408-1417).

However, compared with that in other tissue cells, CD38 antigen was expressed at the highest level in multiple myeloma (MM) and B-lymphoma. Therefore, CD38 antigen has been considered as an ideal target for the treatment of multiple myeloma and B-lymphoma since the early 1990s, and there have been many studies and reports on the successively treatment of multiple myeloma and B-lymphoma in animals in vivo or in vitro with monoclonal antibodies targeting CD38 antigen.

Stevenson FK et al. reported in 1991 that a genetically engineered human-mouse chimeric antibody OKT10 can kill CD38 positive lymphoma cells in vitro through an antibody-dependent cellular cytotoxicity (ADCC) (Stevenson FK et al., Blood. 1991, 7:1071-1079).

Goldmacher VS et al. reported in 1994 that an antibody-drug conjugate, in which an immunotoxin ricin was coupled to a mouse anti-human CD38 monoclonal antibody HB7, exhibited a strong in vitro killing activity against CD38 positive tumor cells (Goldmacher VS et al., Blood. 1994, 84:3017-25).

Ellis JH et al. reported in 1995 that another genetically engineered and humanized antibody from a mouse anti-CD38 monoclonal antibody AT13/5 had a strong ADCC activity than its predecessor (Ellis JH et al., J Immunol. 1995, 155:925-37).

However, these early findings have not subsequently or successfully transformed into useful clinical applications or entered into clinical studies.

Multiple myeloma is a malignant plasma cell disease characterized by the latent accumulation of secretory plasma cells of a low proliferation and an extended long life-span in the bone marrow. The disease can cause the symptoms such as hypercalcemia, renal impairment, destruction of adjacent bone marrow tissue, and anemia. Before 2015, the main therapies for multiple myeloma include chemotherapy drugs such as Vincristine, Cyclophosphamide, Melphalan, Adriamycin, and Immunomodulators (IMiDs), Proteasome Inhibitors (PIs), Bisphosphonates, hormonetherapy such as Prednisone, Dexamethasone, and autologous stem cell transplantation.

Among them, the representative Immunomodulator drugs are Thalomid (generic name: Thalidomide, approved by the U.S. FDA in July 1998), Revlimid (generic name: lenalidomide, approved by the U.S. FDA in December 2005), and Pomalyst (generic name: Pomalidomide, approved by the U.S. FDA in February 2013), all developed and launched successively by Celgene, USA.

The representative proteasome inhibitor drugs are Velcade (generic name: Bortezomib, approved by the U.S. FDA in May 2003) developed by Takeda/Millennium, Japan, and Kyprolis (generic name: Carfilzomib, approved by the U.S. FDA in July 2012) developed by Onyx, USA. The representative bisphosphonates drugs are Aredia (generic name: Pamidronate, approved by the U.S. FDA in October 1991) and Zometa (generic name: Zoledronic, approved by the U.S. FDA in August 2001) successively developed by Novartis.

The complete response rate (CRR) of the combination of these drugs is generally only 5%, the median survival time (MST) of patients is generally 36-48 months after their first diagnosis. Therefore, new and more effective drugs for the treatment of multiple myeloma are urgently being sought.

In November 2015, the world's first anti-CD38 monoclonal antibody drug Daratumumab was approved by the US FDA for the treatment of multiple myeloma patients who have been received at least three rounds of the treatments, including Bortezomib, Lenalidomide or both at the same time and developing resistance to multiple-drugs. The field of the treatment of multiple myeloma then moved into a new era of revolutionary significance, that is monoclonal antibody targeted therapy.

Daratumumab (brand name Darzalex) was developed by Janssen Biotech, a subsidiary of Johnson & Johnson in the US, and Genmab of Denmark. Daratumumab is a fully human IgG1/kappa monoclonal antibody, specifically recognizing and binding human CD38 antigen. It was originally derived from a human CD38 monoclonal antibody code-named 005 developed by Genmab (de Weers et al. J Immunol 2001; 186: 1840-8 ; PCT application No.: PCT/DK2006/000166, US Patent No. :
US2009/0148449A1). Preliminary non-clinical study results showed that Daratumumab/005 could rapidly kill CD38 antigen-positive tumors such as myelomas by multiple mechanisms, including complement-dependent cytotoxicity (CDC), antibody-dependent cell-mediated cytotoxicity (ADCC), antibody-dependent cell-mediated phagocytosis (ADCP), and apoptosis. In addition, Daratumumab/005 can also inhibit ADP-ribosyl cyclase activity mediated by CD38 and thus play a role in efficacy.

In 2011, Johnson & Johnson and Genmab reached an agreement of clinical cooperation and development of Daratumumab. In 2013, Daratumumab achieved a Fast Track Designation and Breakthrough Therapy Designation by the U.S. FDA because of its good single-agent activity demonstrated in a clinical trial for the treatment of myeloma. In November, 2015, Daratumumab was quickly approved for the marketing by the U.S. FDA based on the results of two phase I/II clinical trials, named as GEN501 (Lokhorst HM et al., N Engl J Med. 2015, 373:1207-19) and SIRIUS (Lonial S et al., Lancet. 2016, 387:1551-60), and used as a fourth-line therapy for multiple myeloma patients who have received at least three times of treatments including Bortezomib, Lenalidomide or both at the same time and development of multi-drug resistance. In the GEN501 and SIRIUS clinical trials, the results demonstrated that the objective response rate (ORR) and the median progression-free survival (PFS) in the Daratumumab treatment group (infusion dose of 16mg/kg body weight) were significantly improved.

After Daratumumab's launching, two Phase III clinical studies named as POLLUX and CASTOR were conducted by Johnson & Johnson /Genmab. Wherein POLLUX was to evaluate the clinical efficacy of the combination of Daratumumab with Lenalidomide, and Dexamethasone as a third-line therapy for the treatment of myeloma. A total of 569 myeloma patients were enrolled in POLLUX study, including 286 patients in the group of the combination of Daratumumab, Lenalidomide, and Dexamethasone (treatment group), and 283 patients in the group of the combination of Lenalidomide and Dexamethasone (control group). The study results showed that proportion of patients with progression-free survival (PFS) in the treatment combined with Daratumumab group at 12 months was 83.2%, and the ORR was 92.9%, both are significantly higher than the control group (PFS proportion was 60.1%, ORR was 76.4%) (Dimopoulos et al. POLLUX Investigators, N Engl J Med. 2016, 375:1319-31).

CASTOR was to evaluate the clinical efficacy of the combination of Daratumumab, Bortezomib, and Dexamethasone as a third-line therapy for the treatment of myeloma. A total of 498 myeloma patients were enrolled in this study, including 251 patients in the combination group of Daratumumab, Lenalidomide, and Dexamethasone (treatment group), and 247 patients in the combination group of Bortezomib and Dexamethasone (control group). The study results showed that the proportion of patients with progression-free survival (PFS) in the Daratumumab combined treatment group at 12 months was 60.7%, and the ORR was 82.9%, both are significantly higher than the control group (PFS proportion was 26.9%, ORR was 63.2%) (Palumbo A et al., CASTOR Investigators. N Engl J Med. 2016, 375:754-66).

In November 2016, a combination of Daratumumab, lenalidomide, and Dexamethasone, or a combination of Daratumumab, Bortezomib, and Dexamethasone was approved by the U.S. FDA as a third-line therapy for the treatment of myeloma based on the results from the above two Phase III clinical trials POLLUX and CASTOR.

In June 2017, a combination of Daratumumab, Pomalidomide, and Dexamethasone was approved by the U.S. FDA as a second-line therapy for the treatment of myeloma based on the results in a clinical trial code-named as EQUULEUS(MMY1001). This study showed that the ORR was 60%, the median PFS was 8.8 months, and the median overall survival was 17.5 months in the treatment group of a combination of Daratumumab, Pomalidomide, and Dexamethasone (Ajai Chari, et al, EQUULEUS; MMY1001 Investigators: Blood. 2017, 130: 974-981).

US2018/117150A1 discloses combination therapies comprising an anti-CD38 antibody and cyclophosphamide for CD38-positive hematological malignancies. It discloses anti-CD38 antibodies binding to the same regions of the CD38 as daratumumab.

Based on the results from a clinical trial named as ALCYONE, a combination of Daratumumab, Bortezomib, Melphalan, and Prednisone was approved by the U.S. FDA for the first-line treatment of newly diagnosed myeloma patients who have not received high-dose chemotherapy and autologous stem cell transplantation (ASCT). A total of 706 myeloma patients were enrolled in ALCYONE study, and the results showed that that the proportion of patients with PFS in the Daratumumab combined treatment group at 18 months was 71.6%, the ORR was 90.9%, both significantly higher than the control group (PFS proportion was 50.2%, ORR was 73.9%, Mateos M-V et al., ALCYONE Investigators. N Engl J Med. 2018, 378:518-528).

As a result, in just three years after its first approved by the U.S. FDA in November 2015, Daratumumab has been gradually promoted from a fourth-line, single-agent therapeutic drug to a first-line therapeutic drug, and quickly became a best-selling blockbuster drug on the market. Daratumumab is the only anti-CD38 monoclonal antibody world-wide that has been approved for marketing. The other anti-CD38 monoclonal antibodies currently entered into the clinical trials are SAR650984 (Isatuximab), a human-mouse chimeric antibody developed by Sanofi /Immungen, and MOR202, a fully human monoclonal antibody screened from the phage-display antibody library, developed by MorphoSys, a German pharmaceutical company. The main clinical indications for these antibodies are also myelomas.

In China, the mainly available therapeutic drugs for myelomas are imported Bortezomib/Velcade, lenalidomide, etc. With the patent expiration of Bortezomib/Velcade and other drugs, dozens of domestic enterprises have been developing their generic drugs. Among them, Bortezomib generic drugs developed by Qilu Pharmaceutical, Chia Tai Tianqing, and Jiangsu Hansoh Pharma, have recently been approved by the China Food and Drug Administration (CFDA) to launch for the treatment of myeloma. However, in the CD38-targetingmonoclonal antibody drug areas, only Johnson & Johnson's Daratumumab, and Sanofi's SAR650984 (Isatuximab), both are imported products, have either completed an application for clinical trial or just entered into the clinical trial in China. So far, no CD38 monoclonal antibody developed by domestic enterprises has entered the stage of an application for clinical trial

Because of the large number of myeloma patients at home and abroad, there is a severe shortage of drugs, especially antibody drugs, for the treatment of myeloma. Therefore, it is of great significance and necessity to research and develop new drugs, especially CD38 antigen targeting monoclonal antibodies for the treatment of myeloma,

Although Daratumumab has been widely praised by clinicians, patients and the market since its launching, it still has many defects and deficiencies, including at least the following:
1) When patients receive intravenous infusion of Daratumumab, there is a high rate of clinical adverse reactions, and the administration time is relatively long. For example, the first intravenous infusion takes 8 hours, and the second infusion also takes 5-6 hours, and patients need to receive the treatment at a frequency of once a week during the first 8-week treatment course;
2) Some myeloma patients had no clinical response to Daratumumab treatment; Others continued to progress or developed resistance after the treatment with Daratumumab. The causes and mechanisms of patient' resistance or failure to Daratumumab treatment are not yet known.
3) Daratumumab does not recognize monkey CD38 antigen, which limits its use, either as a single drug or in combination with other drugs in the preclinical efficacy, pharmacological toxicology, and other research and development applications and studies in monkeys or other non-human primates,

Based on a 70% identity between the amino acid sequences of mouse CD38 and human CD38, it is speculated in theory that a new monoclonal antibody with a different epitope of CD38 can be prepared or developed with the traditional technique of immunizing mice with antigen protein and hybridoma production. From them, it is expected that a drug with a greater bioactivity or better safety profile than Daratumumab or other CD38 monoclonal antibodies currently at the clinical research stages, may be developed. These new monoclonal antibodies can be used as a single agent, or sequentially or combined with other drugs currently on the market, such as Bortezomib, Lenalidomide, etc. to treat CD38 high expression tumors such as myeloma and lymphoma.

### SUMMARY

A technical problem to be solved in the present invention is to provide a novel antibody or a derivative thereof such as a Fab fragment, a single-chain antibody, etc., with the antigen-binding site/epitope different from Daratumumab, binding human and monkey CD38 antigens, and with the bioactivity capable of in vivo and in vitro killing tumors expressing high level of CD38 antigen.

A second technical problem to be solved in the present invention is to provide a DNA molecule or gene coding the above antibody.

A third technical problem to be solved in the present invention is to provide a pharmaceutical compound or a pharmaceutical composition comprising the above antibody.

A fourth technical problem to be solved in the present invention is to provide a use of the pharmaceutical compound or the pharmaceutical composition comprising the above antibody for the treatment of tumors expressing high levels of CD38 antigen.

A fifth technical problem to be solved in the present invention is to provide a preparation method of the above antibody.

To resolve the above technical problems, the present invention adopts the following technical solutions:
In one aspect, the present invention provides a novel anti-CD38 monoclonal antibody or a derivative thereof, with antigen-binding site/epitope different from Daratumumab. The monoclonal antibody or the derivative thereof comprises a first variable region and a second variable region, wherein the first variable region is an antibody light chain variable region comprising antigen complementarity-determining regions CDR1, CDR2 and CDR3 having amino acid sequences as set forth in SEQ ID NO : 3, SEQ ID NO : 4 and SEQ ID NO : 5, respectively; and wherein the second variable region is an antibody heavy chain variable region comprising antigen complementarity-determining regions CDR1, CDR2 and CDR3 having amino acid sequences as set forth in SEQ ID NO : 8, SEQ ID NO : 9 and SEQ ID NO : 10, respectively.

The antibodies include a human-mouse chimeric antibody, a half chimeric/half humanized antibody, and a humanized monoclonal antibody; the derivatives include a Fab fragment of an antibody, a single-chain Fab fragment, an Fv fragment, a single-chain antibody, a bispecific antibody, an antibody-drug conjugate (ADC), chimeric antigen receptor T-cell (CAR-T), etc.

As a preferred technical solution, the first variable region is an antibody light chain variable region having an amino acid sequence as set forth in SEQ ID NO : 2; and the second variable region is an antibody heavy chain variable region having an amino acid sequence as set forth in SEQ ID NO: 7.

As a preferred technical solution, the first variable region is an antibody light chain variable region having an amino acid sequence as set forth in SEQ ID NO : 11; and the second variable region is an antibody heavy chain variable region having an amino acid sequence as set forth in SEQ ID NO : 12 or SEQ ID NO: 7.

As a preferred technical solution, the antibody or derivative thereof comprises the antibody light chain variable region, a human antibody light chain constant region, the antibody heavy chain variable region, and a hinge region of a human antibody heavy chain constant region, CH1 region, CH2 region, and CH3 region.

As a preferred technical solution, the human antibody light chain constant region is a kappa chain or a lambda chain of a human antibody; the human antibody heavy chain constant region is a human IgG1, IgG2, IgG3, and IgG4 isotype; wherein the IgG1 isotype is more preferred.

In a second aspect, the present invention provides a DNA molecule or gene coding the above antibody or derivative thereof, wherein the nucleotide sequence of the antibody light chain variable region is set forth in SEQ ID NO : 1 or SEQ ID NO : 13, and the nucleotide sequence of the antibody heavy chain variable region is set forth in SEQ ID NO : 6 or SEQ ID NO : 14.

In a third aspect, the present invention provides an expression vector comprising a nucleotide sequence coding the DNA molecular/gene of the above antibody or derivative thereof and an expression regulatory sequence operably linked to the nucleotide sequence.

In a fourth aspect, the present invention provides a recombinant host cell transfected with the above expression vector. The recombinant host cell or a progeny cell thereof expresses the above antibody or derivative thereof. The antibodies include a humanized monoclonal antibody; the derivatives comprise a Fab fragment of an antibody, a single-chain antibody, a bispecific antibody, etc.

In a fifth aspect, the present invention provides a pharmaceutical compound or a pharmaceutical composition for use in the treatment of tumors, particularly for the tumors with positive CD38 expression. The preferred tumors with positive CD38 expression include human myeloma, human lymphoma (B-lymphoma), etc. A specific example of the present invention depicts a use of the antibody in vivo in inhibiting the growth of high-level CD38 expressing human B-lymphoma Raji cells a with high level of CD38 expression .

As another preferred technical solution, the anti-CD38 antibody in the present invention used for the treatment of CD38 positive tumors can be wild-type or genetically engineered version of the constant regions of human IgG1 or IgM isotype antibody, to maintain or increase antibody-dependent cellular cytotoxicity (ADCC) or complement-dependent cytotoxicity (CDC), to further achieve an effect of a stronger killing of tumor tissues or cells. The wild-type or genetically engineered constant regions of human IgG1 or IgM isotype antibody can be cloned or synthesized in vitro, respectively, by genetic engineering technology known to the skilled in the art.

The CD38 antibody or the derivative thereof in the present invention can be used as a targeting carrier to conjugate or couple with other anti-tumor drugs or toxins to form antibody-drug conjugates (ADC), to targete the tumor tissues and to achieve a better effect of killing tumor. The methods of conjugating or coupling antibody with drugs or toxins can be those of the conventional techniques known to people in this art.

As another preferred technical solution, the anti-CD38 antibody or derivative thereof in the present invention can be administered sequentially or in combination with anti-tumor angiogenesis drugs or inhibitory immune checkpoint molecules to treat tumors with positive CD38 expression.

Wherein the preferred anti-tumor angiogenesis drugs administered sequentially or in combination with the anti-CD38 antibody or derivative thereof in the present invention were the macromolecular biological drugs targeting vascular endothelial growth factor (VEGF) or its receptor (VEGF-R) or small molecular chemical drugs. The preferred targeting VEGF and/or VEGF-R macromolecular biological drugs comprise anti-VEGF monoclonal antibody Bevacizumab (brand name: Avastin), anti-VEGF monoclonal antibody Fab fragment Ranibizumab (brand name: Lucentis); anti-VEGFR2 monoclonal antibody Ramucirumab (brand name: Gyramza) and anti-VEGF monoclonal antibody code-named hPV19 (under development in Suzhou Stainwei Biotech Inc., see Chinese patent document: ZL 201210540692X, patent title: monoclonal antibody for antagonizing and inhibiting the binding of vascular endothelial growth factor to its receptor, as well as coding sequence and use; American granted patent document: US2016/0039921A1); VEGFR-Fc fusion protein drugs such as Albercept (brand name: Eylea), Conbercept, etc. The preferred targeting VEGFR small molecular chemical drugs comprise Sunitinib, Sorafenib, Apatinib, Pazopanib, etc.

The preferred target inhibitory immune checkpoint molecules used sequentially or in combination with anti-CD38 antibody or the derivative thereof in the present invention include anti-CTLA4 (Cytotoxic T-lymphocyte Antigen-4) monoclonal antibody Ipilimumab (brand name: Yervoy); anti-PD-1(programmed death protein-1) antibody Nivolumab (brand name: Opdivo), Pembrolizumab (brand name: Keytruda), code-named hAB21(under development in Suzhou Stainwei Biotech Inc., See PCT patent application document: PCT/CN2017/089282, monoclonal antibody antagonizing and inhibiting binding between human PD-1 antigen and ligand thereof, preparation method thereof and application thereof); anti-PD-L1 monoclonal antibody drugs include Atezolizumab (brand name: Tecentriq), Avelumab (brand name: Bavencio), Durvalumab (brand name: Imfinzi), etc.

As another preferred technical solution, the anti-CD38 antibody in the present invention can be firstly prepared into chimeric antigen receptor T-cell (CAR-T), then introduced into the immune cells isolated from peripheral blood of tumor patients, such as T-lymphocytes, after culturing and amplification in vitro, these lymphocytes recognizing CD38 antigen were injected back into the body to achieve the effect of treating the tumor by targeting tumors with CD38 high expression. The preparation of anti-CD38 antibody in the present invention into chimeric antigen receptor T-cell (CAR-T) can take conventional techniques known to a person skilled in the art.

In a sixth aspect, the present invention provides a method for preparing the above antibody or derivative thereof, and the method comprises the following steps:
a) providing an expression vector comprising the above DNA sequence and an expression regulatory sequence operably linked to the DNA sequence;
b) transfecting a host cell with the expression vector of step a);
c) culturing the host cell from step b) under conditions suitable for the expression of the antibody; and
d) isolating, purifying, and collecting the antibody from a host cell culture medium.

The term "monoclonal antibody (mAb)" used herein refers to an immunoglobin obtained from a clonal cell, with the same structure and chemical characteristics and specific to a single antigenic determinant. The monoclonal antibody is different from a regular polyclonal antibody preparation (usually having different antibodies directed against different determinants). Each monoclonal antibody is directed against a single determinant of an antigen. In addition to its specificity, the monoclonal antibody is also advantageous because it is cultured from hybridoma or recombinant engineering cells and will not be mixed with other immunoglobulins. The modifier "monoclonal" indicates that the antibody's properties are achieved from a homogeneous population of antibodies, which should not be interpreted as any special method that needs to be used for production of antibodies.

The term "humanized monoclonal antibody" as used herein refers to that all or most of the amino acid sequences of the murine monoclonal antibodies (including the framework region sequence in the variable region), except complementarity-determining regions (CDR) are substituted by the amino acid sequences of human immunoglobulins, to reduce the immunogenicity of the murine monoclonal antibody to the utmost extent by genetic engineering methods.

The terms "antibody" and "immunoglobulin" used herein refer to an iso-tetra proteoglycan of about 150,000 Daltons with the same structural characteristics and consist of two identical light chains (L) and two identical heavy chains (H). Each light chain is linked to the heavy chain through a covalent disulfide bond, while the same isotype heavy chains of the different immunoglobulins have a different amount of disulfide bonds. Each heavy chain and each light chain also have regularly spaced intrachain disulfide bonds. Each heavy chain has a variable region (V_{H}) at one end, followed by several constant regions. Each light chain has a variable region (V_{L}) at one end, and a constant region at the other end. The constant region of the light chain is opposite to the first constant region of the heavy chain. The variable region of the light chain is opposite to the variable region of the heavy chain. Special amino acid residues form an interface between the variable region of the light chain and the heavy chain.

The term "variable" used herein indicates that some portion of the variable region in an antibody are different in sequence, which results in binding and specificity of various specific antibodies to the specific antigens. However, variability is not evenly distributed throughout the whole antibody variable region. Instead, it concentrates on three fragments in the complementarity-determining region (CDR) and hypervariable region in the light-chain or heavy-chain variable regions. The more conservative part of the variable region is called the framework regions (FR). There are four FR regions in each variable region of the heavy-chain and light-chain of an antibody. The FR regions are roughly in a β-folded configuration and connected by three CDRs forming a connecting loop. The partial β-folded configuration can form in some cases. The CDRs in each chain are close together through the FR regions and form the antigen-binding site of the antibody together with the CDRs of another chain (see Kabat et al, NIH Publ. No. 91-3242, Vol. 1, pp. 647-669 (1991)). The antibody's constant region does not directly participate in the binding of the antibody to the antigen. Still, it exhibits different effects and functions, such as participating in antibody-dependent cellular cytotoxicity (ADCC) and complement- dependent cytotoxicity (CDC) of the antibody.

The antibody of the present invention can be usually prepared by the following methods:
Firstly, insert the gene coding the antibody in the present invention into the expression vector containing a suitable expression regulatory sequence.

The term "expression regulatory sequence" used herein usually refers to a sequence that participates in the control of the gene expression. The expression regulatory sequence includes a promoter operable linked to the target gene and a termination signal. The gene (DNA) sequence of the present invention's antibody can be encoded by the common techniques well known by the skilled in the art, such as artificial synthesis according to the protein sequences disclosed by the present invention or the PCR amplification. After that, the DNA fragments synthesized or amplified by the PCR method can be inserted into a suitable expression vector by various methods well known in the art. The expression vector used in the present invention can be available on the market and well known for those skilled in the art, such as the pCDNA3.1 expression vector from Invitrogen.

The suitable host cells for accepting the expression vector transformation generally include both prokaryotes and eukaryotes. Commonly used prokaryotes host cells include *E*. *coli,* and *Bacillus subtillis,* etc. Commonly used eukaryotes host cells include yeast cells, insect cells, and mammalian cells. In the present invention, the preferred host cells are mammalian, particularly Chinese hamster ovary (CHO) cells.

The host cells transfected by the expression vector are cultured under suitable conditions (e.g., culturing with a serum-free culture medium in a cell culture flask or bioreactor by adhesion to the wall or suspension). The supernatant is collected and purified by common separation steps or means well known by the skilled in the art, including protein-A affinity chromatography, ion-exchange chromatography, filtration, etc. to produce the antibodies of the present invention.

The purified antibodies of the present invention can be dissolved in an appropriate solvent such as sterile saline liquid. The concentration can be prepared between 0.01 and 100 mg/mL. The ideal final concentration can be prepared between 1 mg/ml and 20 mg/ml.

To obtain novel murine monoclonal antibodies specifically binding CD38 antigen as well as the hybridoma cell lines secreting these antibodies, the present invention chose recombinant human CD38 protein extracellular domain expressed by the mammalian cell as an immune antigen, then immunized mice to obtain the anti-CD38 polyclonal antibodies by repeated, small dose subcutaneous injections. The mice with high titers of antibodies were selected to prepare the spleen cells, fused the spleen cells with a mouse myeloma cell line in vitro. After a few steps including drug selection and subcloning, several hybridoma monoclonal cell lines stably secreting anti-human CD38 antibodies were established. A mouse hybridoma clone, named as m29, was found by ELISA, flow-cytometry and other methods that it secreted an monoclonal antibody not only specifically binding to human CD38 protein as well as several CD38 positive human tumor cell lines, but also capable of killing the CD38 positive tumor cells by CDC in vitro.

The gene fragments coding the heavy-chain and light-chain variable region were obtained by genetic engineering methods, etc. in the present invention. The expression vectors expressing the human-mouse chimeric antibody or the humanized antibody were obtained after genetic engineering. The expression vectors were transfected into Chinese hamster ovary (CHO) cells to obtain the engineering cells stably and efficiently secreting the human-mouse chimeric antibody or the humanized antibody. The culture supernatants from the engineering cells were isolated and used to purify either the human-mouse chimeric antibody ch29 protein or the humanized antibody HH29 protein.

Characterization analysis by many in vitro methods, including competitive ELISA, flow cytometry, etc. showed thatthe CD38 antigen binding epitopes of the murine monoclonal antibody m29 and its human-mouse chimeric antibody ch29 to are different from that of Daratumumab. The analysis results in vitro by direct ELISA, flow cytometry, etc. also showed that the murine monoclonal antibody m29 and the human-mouse chimeric antibody thereof ch29 can specifically bind to the recombinant monkey CD38 protein and CHO cell lines expressing monkey CD38 gene with a high affinity; in contrast, the monoclonal antibody Daratumumab only binds to human CD38 antigen but not to monkey CD38 antigen.

The anti-tumor effects of the murine monoclonal antibody m29 and the human-mouse chimeric antibody ch29 were tested in immunodeficient mice in vivo. The results showed that in vivo administration of the murine monoclonal antibody m29 or the human-mouse chimeric antibody ch29 thereof after had obvious inhibition to the tumor growth, with a efficacy that is no less than the positive control drug such as Rituximab (brand name: Rituxan, a human-mouse chimeric anti-CD20 monoclonal antibody) or Daratumumab.

### BRIEF DESCRIPTION OF THE DRAWINGS

Fig. 1 is a schematic diagram of the comparison analysis of the amino acid sequences of human CD38 protein (SEQ ID NO: 24) and mouse CD38 protein (SEQ ID NO: 25) in Example 1 of the present invention.
Fig. 2A is a schematic diagram of the OD values from various hybridoma cell line samples screened for secreting anti-CD38 antibody by ELISA in Example 1 of the present invention.
Fig. 2B is a schematic diagram of the CDC assay results: supernatant samples from anti-CD38 antibody secreting hybridoma cell lines were evaluated for their capability of killing Daudi target cells in vitro using a CDC method in Example 2 of the present invention; wherein the target cells are Daudi cells, RFU is an abbreviation of Relative Fluorescence Unit.
Fig. 3A is a schematic diagram of the CDC activity of the supernatant from mouse hybridoma cell line m29 analyzed by CDC method in Example 3 of the present invention; wherein the target cells are Daudi, the positive control sample is Daratumumab, and the negative control sample is the supernatant from a mouse SP2/0 myeloma cell line.
Fig. 3B is a schematic diagram of the CDC assay result of the positive control sample Daratumumab in Example 3 of the present invention.
Fig. 3C is a schematic diagram of the CDC assay result of the supernatant sample of the mouse hybridoma cell line m29.
Fig. 4 is schematic diagrams of the representative results in determining and analyzing the binding of the supernatant sample from mouse hybridoma cell line m29 to CD38 positive human tumor cell lines by flow cytometer in Example 4 of the present invention; the negative control sample is a non-related mouse hybridoma mAB21 (mouse anti-human PD-1 monoclonal antibody); wherein
Fig. 4A is a result diagram showing the detection of human B-lymphoma cell line Daudi by flow cytometer;
Fig. 4B is a result diagram showing the detection of human myeloma cell line RPMI-8226 by flow cytometer;
Fig. 4C is a result diagram showing the detection of human T-lymphoma cell line MOLT-4 by flow cytometer;
Fig. 5 is representative result diagrams of determining and analyzing the binding of the mouse monoclonal antibody m29 sample and the positive control sample Daratumumab to CHO cells transfected uman CD38 gene (CHO-hCD38); the negative control sample is the non-related mouse hybridoma mAB21 (mouse anti-human PD-1 monoclonal antibody) or a humanized monoclonal antibody hAB21; Wherein
Fig. 5A is a result diagram of the mouse monoclonal antibody m29 by flow cytometer;
Fig. 5B is a result diagram of the positive control sample Daratumumab by flow cytometer.
Fig. 6 is schematic diagrams of detecting the competitive binding of the purified mouse monoclonal antibody m29 sample and Daratumumab to CD38 by ELISA in Example 6 of the present invention; the negative control sample is the non-related humanized monoclonal antibody sample hPV-19 (anti-VEGF monoclonal antibody); Wherein
Fig. 6A is a result diagram of the competitive binding of mouse monoclonal antibody m29, Daratumumab, and Biotin-labeled Daratumumab sample to CD38;
Fig. 6B is a result diagram of the competitive binding of mouse monoclonal antibody m29, Daratumumab, and Biotin-labeled monoclonal antibody m29 to CD38;
Fig. 7 is the comparison analysis diagrams of the variable region amino acid sequences of the mouse monoclonal antibody m29 and Daratumumab in Example 7 of the present invention, wherein
Fig. 7A is the comparison analysis diagram of a light chain variable region amino acid sequences; the different amino acids between m29 and Daratumumab (SEQ ID NO: 26) are marked by "X", the amino acid sequence areas of CDR1, CDR2, and CDR3 of the light chain variable region are marked in box.
Fig. 7B is the comparison analysis diagram of a heavy chain variable region amino acid sequences; the different amino acids between m29 and Daratumumab (SEQ ID NO: 27) are marked by "X", the amino acid sequence areas of CDR1, CDR2, and CDR3 of the heavy chain variable region are marked in box.
Fig. 8 is the representative result diagrams of determining and analyzing the binding of the mouse monoclonal antibody m29 sample, the human-mouse chimeric antibody ch29G, and the positive control sample Daratumumab or Rituximab (Rituxan, a human-mouse chimeric anti-CD20 monoclonal antibody) to human tumor cell lines; wherein
Fig. 8A is a result diagram showing the detection of human B-lymphoma cell line Raji by flow cytometer;
Fig. 8B is a result diagram showing the detection of human myeloma cell line RPMI-8226 by flow cytometer;
Fig. 8C is a result diagram showing the detection of human T-lymphoma cell line MOLT-4 by flow cytometer;
Fig. 8D is a result diagram showing the detection of human T-lymphoma cell line Jurkat by flow cytometer;
Fig. 9 is the diagram of the CDC assay of detecting and analyzing the mouse monoclonal antibody m29 sample, the human-mouse chimeric antibody ch29G sample, and the control sample Daratumumab, wherein the target cell used is human B-lymphoma cell line Daudi, the source of complement is the rabbit serum, and negative control sample is a non-related humanized hPV19 Mab (anti-VEGF Mab).

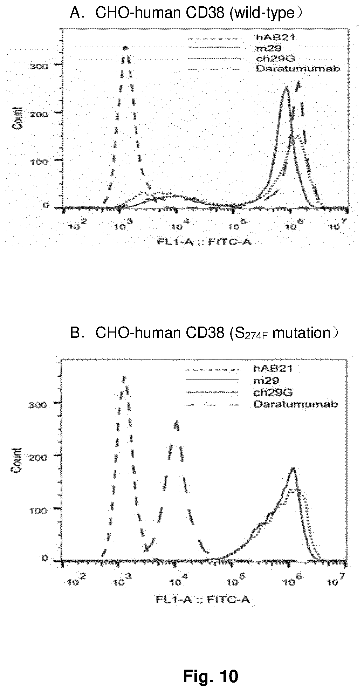

Fig. 10 is the diagram of the binding of the mouse monoclonal antibody m29 sample, the human-mouse chimeric antibody ch29G sample, and the control sample Daratumumab to CHO cells stably transfected and expressed with wild-type human CD38 (CHO-hCD38/wild-type) or CHO cells stably transfected and expressed with CD38 with S_{274F} mutation (CHO-hCD38/S_{274F} mutation) in Example 11 of the present invention; the negative control sample is a non-related humanized monoclonal antibody hAB21 (anti-human PD-1 monoclonal antibody). Wherein Fig. 10A is a detecting result diagram of CHO cell lines transfected and expressed with wild-type human CD38; Fig. 10B is a detecting result diagram of CHO cell line stransfected and expressed with human CD38 with a point mutation (CHO/hCD38-S_{274F});
Fig. 11A is the comparison analysis of the amino acid sequences of human CD38 protein (SEQ ID NO: 28), chimpanzee CD38 protein (SEQ ID NO: 29), and cynomolgus macaque CD38 protein (SEQ ID NO: 30).
Fig. 11B is the diagram of the binding of the mouse monoclonal antibody m29 sample, the human-mouse chimeric antibody ch29G, and the control sample Daratumumab to CHO cells stably transfected and expressed with cynomolgus macaque CD38 (CHO-cynomolgus CD38) in Example 12 of the present invention; wherein the negative control sample is the non-related humanized monoclonal antibody hAB21 (anti-human PD-1 monoclonal antibody).
Fig. 12 is the diagram of the detecting and comparing the binding of the human-mouse chimeric antibody ch29G sample and Daratumumab to recombinant human CD38 protein (Fig. 12A) and recombinant cynomolgus macaque CD38 protein (Fig. 12B) in Example 13 of the present invention; wherein the negative control sample is Rituximab (brand name: Rituxan, the human-mouse chimeric anti-CD20 monoclonal antibody).
Fig. 13 is the diagram of CDC activity in the human-mouse chimeric antibody ch29G sample, Daratumumab, and the positive control sample Rituximab (human-mouse chimeric anti-CD20 monoclonal antibody); the source of complement is human serum, and the negative control sample is a non-related humanized hPV19 Mab (anti-VEGF Mab) in Example 14 of the present invention, wherein
Fig. 13A is the diagram of CDC assay targeting human B-lymphoma cell line Daudi;
Fig. 13B is the diagram of CDC assay targeting human B-lymphoma cell line Raji;
Fig. 13C is the diagram of CDC assay targeting human T-lymphoma cell line MOLT-4;
Fig. 13D is the diagram of CDC assay targeting human T-lymphoma cell line Jurkat.
Fig. 14 is the diagram showing in vivo anti-tumor efficacy of the mouse monoclonal antibody m29 in Raji tumor model inoculating human B-lymphoma cells subcutaneously in nude mouse in Example 17 of the present invention; wherein Fig. 14A is a schematic diagram of average tumor growth volume ten days before inoculating; Fig. 14B is a schematic diagram of the average growth volume of the tumor at the later stage of inoculating.
Fig. 15 is the diagram showing the in-vivo anti-tumor efficacy result of the human-mouse chimeric antibody ch29G in Raji tumor model inoculating human B-lymphoma cells subcutaneously in nude mouse in Example 18 of the present invention.

### DETAILED DESCRIPTION OF THE EMBODIMENTS

The present invention will be further described by reference to the following examples, which are illustrative only and are not intended to limit the present invention.

Tumor cell lines and DNA primers used in the present invention are shown in Table 1 and Table 2, respectively:

**Table 1 : Names and characteristics of tumor cell lines used in the present invention**

| Names of cell lines (ATCC code-name) | Origin of tumors | Surface antigen characteristics | Main references |
|---|---|---|---|
| Daudi (CCL-213) | B-lymphoblast, | CD20⁺, CD38⁺, sIgM⁺ | Klein E, et al. Surface IgM-kappa specificity on a Burkitt lymphoma cells. Cancer Res. 28: 1300, 1968 |
| | Burkitt's lymphoma patients | | |
| Raji (CCL-86) | B-lymphoblast, | CD38⁺, CD20⁺, sIgM⁻ | Pulvertaft JV. Cytology of Burkitt's tumour (African lymphoma) Lancet 1: 238-240, 1964 |
| | Burkitt's lymphoma patients | | |
| RPMI8226 (CCL-155) | B-lymphoblast, | CD19,CD20, CD28⁺,CD38⁺,CD49⁺ sIgM⁻ | Matsuoka Y, et al. Proc. Soc. Exp. Biol. Med. 125: 1246-1250, 1967 |
| | myeloma patients | | |
| Molt-4 (CCL-1582) | T-lmphoblast, | CD3A (26%), CD3B | Minowada J, et al. J. Natl. Cancer Inst. 49: 891-895, 1972 |
| | acute lymphoblastic leukemia patients | (33%), CD3C (34%), CD4(55%) | |
| Jurkat (TIB-152) | T-lmphoblast, acute T-cell leukemia patients | CD3⁺, CD4⁺ | Gillis S, Watson J. J. Exp. Med. 152: 1709-1719, 1980 |

**Table 2: Names and sequences of primers used in the present invention**

| **Names of primers** | **Sequences** |
|---|---|
| 1.huCD38F- HindIII-2 | TTGTAAGCTTGCCGCCACCATGGCTAACTGCGAGTTCT CC (SEQ ID NO : 15) |
| 2.huCD38-S274F-R1 | CTTATCGGGCCTATAGATATTTTTGCAGAAGAACTGGA TGTTCCGCTTGCTGATGATGC（SEQ ID NO：16） |
| 3.huCD38-S274F-R2-XhoI | TGGTCTCGAGTCAGATCTCGGAGGTGCAGCTGGAGTCT TCGGGGTTCTTCACGCACTGTAAAAACTTATCGGGCCT ATAGATATT (SEQ ID NO：17) |
| 4.mKaRT | TGTCGTTCACTGCCATCA AT (SEQ ID NO : 18) |
| 5.mGaRT | GCAAGGCTTACAACCACAATC (SEQ ID NO : 19) |
| 6.mIgLF1 | GACATTGTGATGWCMCA (W=A or T, M = A or C) ( SEQ ID NO : 20) |
| 7.mIgLCR440 | CTGAGGCACCTCCAGATGTT (SEQ ID NO : 21) |
| 8.mIgHset1 | CARCTGCARCARYCT (G, R= A or G, Y=C or T) (SEQ ID NO : 22) |
| 9.mIgHCR135 | GTGCTGGAG GGG ACA GTC ACT (SEQ ID NO : 23) |

### Example 1. Establishment and Screening of Mouse Hybridoma Cell Line Secreting Anti-Human CD38 Antibody

### 1.1 Comparison Analysis of the Amino Acid Sequences of Human CD38 Protein and Mouse CD38 Protein.

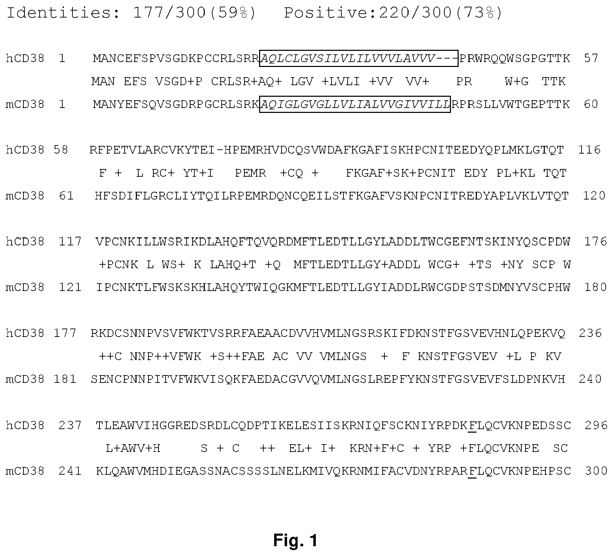

The comparison analysis of amino acid sequences of human CD38 protein and mouse CD38 protein was shown in Fig. 1 (amino acid sequences of the transcellular membrane regions were marked in box and italics). As shown in Fig. 1, there is only 59% identity in the amino acid sequences between human CD38 protein and mouse CD38 protein; therefore, it is speculated that mouse anti-human CD38 monoclonal antibodies targeting different binding regions or amino acid sites can be obtained by the traditional antigen protein immunization in mice coupled with hybridoma preparation techniques. It is expected that the bioactivity and efficacy of these anti-human CD38 monoclonal antibodies, in vitro and in vivo, may be different or even better than those of existing CD38 monoclonal antibodies, such as Daratumumab, due to the different antigen-binding sites/epitopes from the existing CD38 monoclonal antibodies such as Daratumumab. These novel, CD38 antigen new site recognizing monoclonal antibodies scan be used as pharmaceutical components, on the one hand, administered sequentially or combined with the myeloma therapeutic drugs on the market such as Bortezomib, Lenalidomide to enhance the therapeutic efficacy of myeloma; on the other hand, these antibodies are also expected to be developed for the treatment of other CD38 positive tumors such as B-lymphoma, T-lymphoma, etc.

Therefore, the present invention developed and prepared the novel anti-CD38 antibodies; the specific preparation steps were as follows:

### 1.2 Establishment and Screening of Mouse Hybridoma Cell Lines Secreting Anti-CD38 Antibody

### Step 1: Source of recombinant human CD38 protein (immunizing antigen) and animal immunization

In the example of the present invention, immunizing antigen is the recombinant human CD38 extracellular membrane protein expressed by a mammalian cell (Human CD38 Protein-His Tag, Sino Biological Inc. Catalog : 10818-H08H). The recombinant human CD38 protein was mixed with Complete Freund's Adjuvant (Sigma, USA) and injected into Balb/c mice subcutaneously at multi-points (100 µl/mouse, 10 µg CD38 protein each time). Three weeks after the first immunization, CD38 protein was mixed with Incomplete Freund's Adjuvant and immunizing mice subcutaneously at multi-points at a dose of 10 µg/mouse; after that, the mice were boosted two to three times every two weeks in the same way; one week after the third booster immunization, the serum was collected from the mice tail vein blood, then the anti-CD38 antibody titers in mice serum were detected by an ELISA method using 96-well plates coated with the recombinant human CD38 protein.

The ELISA procedures were as follows:
The 96-well plates were coated with the recombinant human CD38 protein (2 µg/mL in a pH 9.6, 0.1 M NaHCOs solution), incubated at 37°C for 2 hours, 2% Bovine Serum Albumin (BSA) was then added and sealed overnight at 4°C. The next day, the coated plates were washed with PBS-0.1% Tween20 solution, followed by the addition of immunized mice serum samples with serial dilutions (an unimmunized mouse serum as a negative control) and incubated at 37°C for 2 hours; after washing with PBS-0.1% Tween20 solution, the HRP-goat anti-mouse IgG antibody (Sigma, USA) was added and incubated at 37°C for 1 hour; after washing with PBS-0.1% Tween20 solution again, the substrate solution O-Phenylenediamine (OPD)-0.1% H₂O₂ was added for a color development for about 10-15 minutes, then 0.1M HCl solution was added to quench the reaction. Thereafter, the OD values at 492 nm were recorded in a multimode reader (PerkinElmer Victor X3). The mouse splenocytes with relatively high titers were collected for the fusion of the next step.

### Step 2: Cell fusion

Three to four days after the last immunization (the fourth booster immunization), spleen were taken out and splenocyte suspensions were prepared in a sterile condition, fused with the mouse SP2/0 myeloma cells (purchased from Cell Center of Shanghai Institute of Life Sciences, Chinese Academy of Sciences) at a ratio of 5:1 or 10: 1 at 50% PEG-1000 (Sigma, USA) based upon standard protocols (Kohler G and Milstein C: Nature 1975; 256:495-497): 1 mL PEG was added slowly within 60 seconds, reacted for 90 seconds, terminated the reaction with the serum-free RPMI-1640 culture medium, centrifuged 10 minutes with 1000 rpm to remove the supernatant; the precipitated cells under the centrifugation were obtained and adjusted the cells density to 1×10⁶/ml with RPMI 1640-10% FCS culture medium containing 10% HAT( H for hypoxanthine, A for aminopterin, T for thymidine nucleoside, Sigma, USA), then added into 96-well flat-bottom cell culture plates (200 ul/well), and incubated in an incubator containing 5% CO2 (Thermo, USA) at 37°C for two to three weeks.

### Step 3: Screening of mouse hybridoma cell lines positively secreting anti-CD38 antibody by ELISA

The same ELISA method to the above detection of anti-CD38 antibody titer in mouse serum was used to detect anti-CD38 antibodies in mouse hybridoma cell culture supernatant.

The ELISA procedures were as follows:
The 96-well plates were coated with the recombinant human CD38 protein (2 µg/mLin a pH 9.6, 0.1 M NaHCOs solution) at 37°C for 2 hours, 2% Bovine Serum Albumin (BSA) was added and sealed overnight at 4°C. The next day, the coated plates were washed with PBS-0.1% Tween20 solution, followed by the addition of the hybridoma cell culture supernatant to be detected (an unfused SP2/0 myeloma cell culture supernatant as a negative control, serum sample of mice immunized with CD38 antigen as a positive control) and incubated at 37°C for 2 hours; after washing with PBS-0.1% Tween20 solution, the HRP-Goat anti-Mouse IgG antibody (Sigma, USA) was added and incubated at 37°C for 1 hour; after washing with PBS-0.1% Tween20 solution again, the substrate solution o-Phenylenediamine (OPD)-0.1% H₂O₂ was added for the development of color for about 10-15 minutes, then 0.1M HCl solution was added to quench the reaction. Thereafter, the OD values at 492 nm were recorded in a multimode reader (PerkinElmer Victor X3).

More than 600 mouse hybridoma cell clones were screened, and more than ten positive clones with anti-CD38 antibody secretion were obtained (the determination criteria of positive clones: the OD value was more than five times higher than that of the negative control sample).

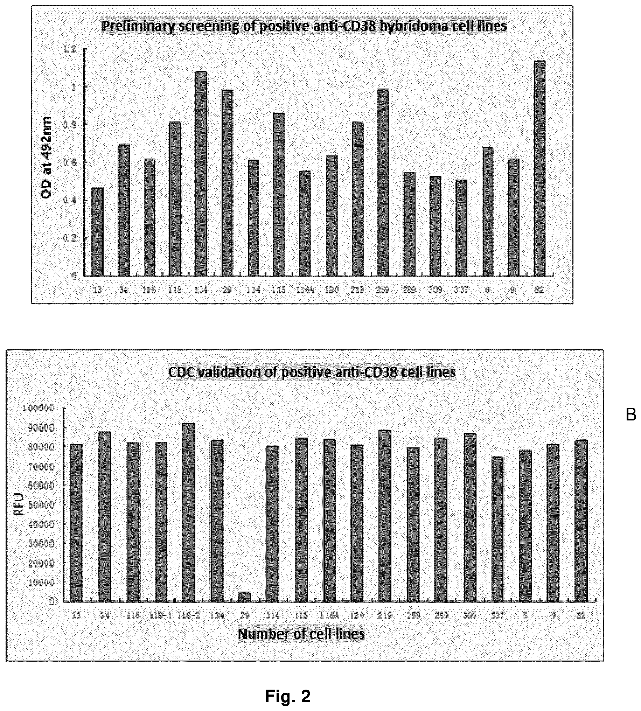

Fig. 2A shows the OD values of various hybridoma cell lines with the positive binding to CD38 antigen screened by ELISA. These positive hybridoma cells were amplified, cultured, and frozen at negative 70 °C.

### Example 2: Screening and assaying hybridoma cell culture supernatant by CDC in Vitro

In the present example, the CDC method used for an in vitro screening and detecting the hybridoma cell culture supernatant with positive anti-CD38 antibody secretion was summarized as follows:

### 2.1 Experimental materials

Cell culture medium: RPMI-1640, Hyclone
Fetal bovine serum (FBS): Gibco, USA
Target cells: human B-lymphoma cell line Daudi (human CD38⁺, CD20⁺, B-lymphoma cell lines or other tumor cell lines, purchased from Cell Bank of Typical Culture Preservation Council, Chinese Academy of Sciences)
Source of complement: human serum/young rabbit serum/rabbit complement (healthy) (in-house)
Samples used for test: hybridoma cell culture supernatants with positive secretion of anti-CD38 antibody (in-house)
Positive control samples: Daratumumab (humanized anti-CD38 monoclonal antibody) or Rituximab (human-mouse chimeric anti-CD20 antibody)
Negative control sample: monoclonal antibody hPV19 (humanized anti-VEGF monoclonal antibody)
Cell Viability Assay Kit: CellTiter-Glo^{®} Luminescent Cell Viability Assay Kit (Promega) 96-well cell culture plates: Coming-3610

### 2.2 Experimental steps:

1) 10 µl of a 5-fold or 3-fold serial diluted antibody samples (the maximum initial concentration of the antibody sample was 20-200 µg/mL) or 10 µl of the hybridoma cell supernatants were directly added into the corresponding wells of the 96-well plates;
2) The target cells in the logarithmic growth phase (Daudi cells or other cells with CD38 antigen positive expression) were collected, washed once with CDC diluent (1% FBS RPMI1640 medium) and counted (the cell viability rate should be more than 90%), and the density of resuspended cells was adjusted to 2.5 × 10⁵/mL;
3) The target cells were added to 96-well plates (Corning-3610), 80 µl/well, about 2×10⁴ cells/well, then incubated the plates in an incubator at 37°C for 30 minutes;
4) Diluted human serum or rabbit serum (first diluted with CDC diluent at 1:10 or 1:20 was added at 10µl/well, then incubated the plates in an incubator at 37°C for 1 to 2 hours;
5) Before adding the Cell Viability kits materials, the cell culture plates and Cell Titer-Glo^{®}Luminescent Cell Viability kits were stayed away from the light and to balanced to the room temperature for 30 minutes. Then 100 uL of the prepared reagent was added into each well, let staining at room temperature and keeping away from light for 10-15 minutes. Then, the RFU (Relative Fluorescence Unit, or Relative Luminescent Unit, RLU) was measured in a multimode reader (PerkinElmer Victor X3).

### 2.3 CDC activity calculation

CDC activity can be directly counted with labeled RFU or the cell lysis rate of the CDC can be calculated according to the following formula:
Cell lysis rate (%)=100 ×( 1-(RFU of sample wells) / ((RFU cells +serum wells))
Cell viability was calculated according to the following formula:
Cell viability (%) =100 × ( RFU sample wells ) / (RFUcells+serum wells))

### 2.4 Results of CDC assay

Fig. 2B shows the representative results of the in vitro killing of the target Daudi cells by supernatants from anti-CD38 antibody hybridoma cell line (RFU direct value) detected by the CDC method; wherein the supernatants from a hybridoma cell line named as m29 showed a significant CDC activity (RFU value decreased by more than 95% compared with other samples); however, the supernatant samples from other hybridoma cell lines did not show significant CDC activities (there was no significant decrease in RFU value).

### Example 3: Identification of the Isotype of the Murine Monoclonal Antibody m29 and Re-validation of CDC Activity Thereof

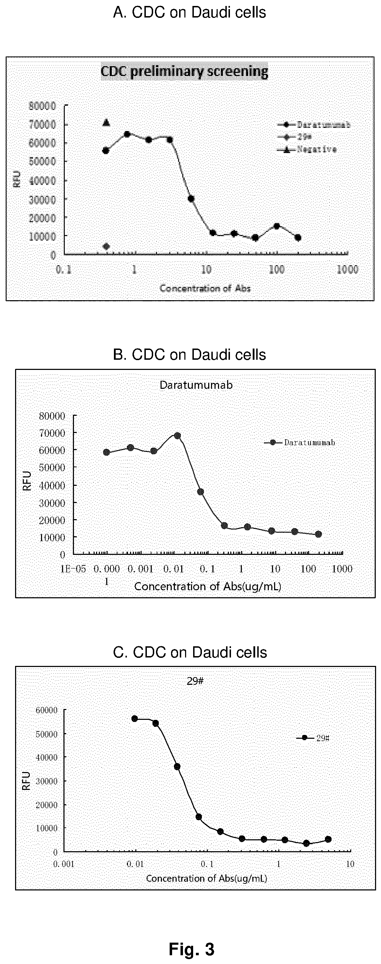

Murine monoclonal antibody m29 was identified as IgG2b isotype using a commercial mouse monoclonal antibody IgG subclass test card (Beijing Biodragon Immunotech Co., LTD., item No. BF06038). After that, the activity of killing target Daudi cells by the hybridoma cell line m29 supernatant was validated by the CDC in vitro; the results were shown in Fig. 3.

Wherein Fig. 3A is the RFU value of hybridoma cell line m29 supernatant sample, which was directly added into CDC testing plates without dilution; the results showed again that the hybridoma cell line m29 supernatant has a significant CDC activity (RFU value decreased by more than 95% compared with negative control);
Fig. 3B is the result of the killing target Daudi cells by the positive control samples Daratumumab of different concentrations by the CDC in vitro; the result showed a significant dose-response curve.

Fig. 3C is the result of the killing target Daudi cells by the monoclonal antibody samples m29 of different concentrations by the CDC in vitro; the result also showed a significant dosage-response curve.

### Example 4: Detecting and Analyzing the Binding of Murine Monoclonal Antibody m29 to Tumor Cell Lines Expressing Human CD38 Antigen by Flow Cytometer

In the present example, the hybridoma cell line m29 supernatant sample of or a non-related murine hybridoma mAB21 sample (mouse anti-human PD-1 monoclonal antibody) were used as a primary antibody, FITC-Goat anti-Mouse IgG was used as a second antibody, the binding of the sample to the human tumor cell lines with positive CD38 antigen expression was detected and analyzed by flow cytometer.

For this purpose, the tumor cell lines with a positive CD38 antigen (B-lymphoma cell line Daudi, myeloma cell line RPMI-8226, and T-lymphoma cell line MOLT-4, purchased from Cell Center of Shanghai Institute of Life Sciences, Chinese Academy of Sciences) respectively were mixed with the hybridoma cell line m29 supernatant sample or a non-related murine hybridoma mAB21 sample (mouse anti-human PD-1 monoclonal antibody) to incubate at 4 °C for 1 hour; after washing with PBS-0.1% FCS solution, FITC-Goat anti-Mouse IgG antibody (diluted at 1:200, Sigma) was added and incubated at 4 °C for 1 hour; after washing with PBS-0.1% FCS solution again, the sample was loaded into Accuri C6 Plus Flow Cytometer (Becton Dickinson, USA; Mountain View, CA, USA).

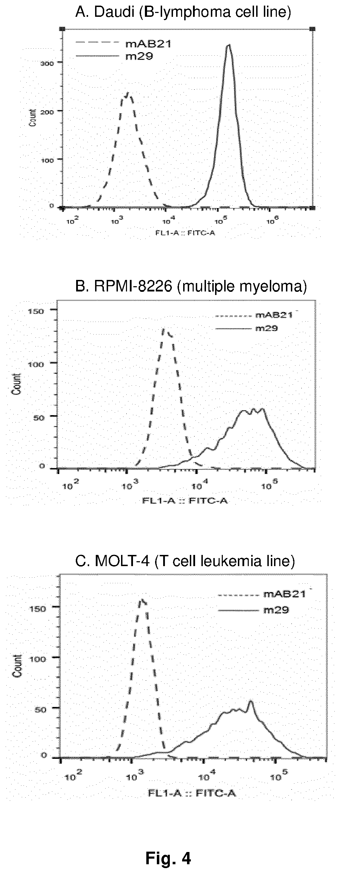

Fig. 4 is the representative flow-cytometry result. As shown in Fig. 4, compared with the non-related murine hybridoma mAB21 sample (mouse anti-human PD-1 monoclonal antibody), the hybridoma cell line m29 supernatant showed significantly binding to CD38 positive tumor cell lines such as human B-lymphoma cell line Daudi (Fig. 4A), myeloma cell line RPMI-8226 (Fig. 4B) and T-lymphoma cell line MOLT-4 (Fig. 4C).

### Example 5: Detecting and Analyzing the Binding of Supernatant Samples of m29 Hybridoma Cell Line or Daratumumab to CHO Cells Stably Transfected with Human CD38 Gene (CHO-hCD38) by Flow Cytometer

### 5.1 Construction of CHO cell lines (CHO-hCD38) stably expressing human CD38 gene

According to human CD38 gene sequences published in Genbank (Gene ID: 952), the CD38 coding cDNA fragments were synthesized by Suzhou Genewiz Biological Technology Co. LTD and then cloned into the cell expression plasmid pQY-DHFR (in-house), transferred into *E.coli,* the recombinant expressing plasmid pQY-DHFR-hCD38 was then identified by restriction endonuclease digestion. After that, the recombinant plasmid pQY-DHFR-hCD38 were mixed Fugen-6 liposome (Roche) and co-transfected into CHO-DHFR⁻ cell, the CHO cell lines stably expressing human CD38 gene (CHO-hCD38)were successfully obtained by screening in IMDM culture medium containing FBS.

### 5.2 Detecting and analyzing the binding of supernatant samples of murine m29 hybridoma cell line or Daratumumab to CHO cells stably expressing human CD38 gene (CHO-hCD38) by flow cytometer

In the present example, the murine m29 hybridoma cell line supernatant sample or the positive sample Daratumumab were used as a primary antibody, FITC-Goat anti-Mouse IgG or FITC-Goat anti-human IgG was used as a second antibody, the binding of the samples to CHO cells stably expressing human CD38 gene (CHO-hCD38) was detected and analyzed by a flow cytometer.

For this purpose, CHO-hCD38 cells were mixed with either the murine m29 hybridoma cell line supernatant sample, the positive sample Daratumumab, the non-related murine hybridoma mAB21 sample (mouse anti-human PD-1 monoclonal antibody), or humanized monoclonal antibody sample hAB21 respectively, and incubated at 4 °C for 1 hour; after washing with PBS-0.1% FCS solution, FITC-Goat anti-Mouse IgG (diluted at 1:200, Sigma) was added (FITC-Goat anti-Human IgG (diluted at 1:200, Sigma) was added if corresponding to Daratumumab sample), and incubated at 4 °C for 1 hour; after washing with PBS-0.1% FCS solution again, the samples were loaded into Accuri C6 Plus Flow Cytometer (Becton Dickinson, USA; Mountain View, CA, USA).

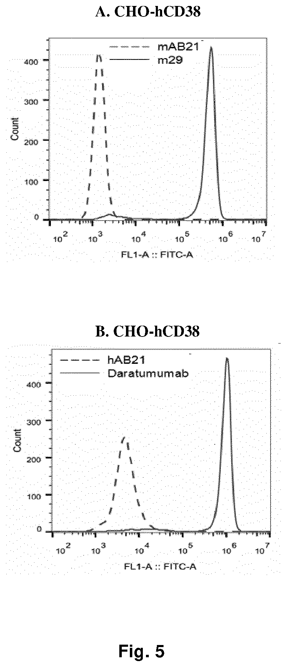

Fig. 5 is the representative flow cytometry analysis results. As shown in Fig. 5, the murine m29 hybridoma cell line supernatant sample (Fig. 5A) and Daratumumab sample (Fig. 5B) showed significantly binding to CHO-hCD38 cells, compared with either the negative control sample, or the non-related murine hybridoma mAB21 or hAB21; wherein the binding intensity of the monoclonal antibody sample m29 to the CHO-hCD38 cell was no less than that of Daratumumab.

### Example 6: Isolation and Purification of Murine Monoclonal Antibody m29 and Detecting the Competitive Binding of Monoclonal Antibody m29 and Daratumumab to CD38

### 6.1: Isolation and purification of murine monoclonal antibody m29

The m29 hybridoma cells were amplified and adapted to culture in serum-free medium (KD-Hybri, Zhuhai Kairui Biotech Co Ltd.). A certain amount of serum-free cell culture supernatants was collected, after centrifugation and filtration with a 0.45 µm filtration membrane, then the supernatants were loaded to a Protein-G affinity chromatography column (Protein G-Sepharose Fast Flow, GE, USA). After rinsing by 1×PBS and eluting with a buffer solution (0.05m sodium acetate, pH=3.2), the m29 antibody was obtained. Thereafter, the buffer was changed to a glycine-Tris (pH=6.0) buffer by a centrifugation with ultrafiltration device (Millipore UFC903096, 30kD), and the purified m29 antibody was stored at 4 °C after quantitative measuring.

### 6.2 Detecting and analyzing the competitive binding of murine monoclonal antibody m29 and Daratumumab to CD38 by a competitive ELISA in vitro

The basic principle and process of the competitive ELISA were as follows: m29 monoclonal antibody samples or Daratumumab with different solubility were mixed with biotin-labeled m29 monoclonal antibody sample (biotin-m29) or biotin-labeled Daratumumab (biotin-Daratumumab), then the mixture was transferred to 96-well plates pre-coated with CD38-His recombinant protein, incubated and eluted, enzyme-labeled Avidin (HRP-Avidin) was added; incubated and eluted again, the substrate was added for staining and detecting the OD values.

In the present example, the detailed steps of the competitive ELISA method were as follows:
1) The 96-well cell culture plates were coated (coating concentration: 2 µg/ml, 50 µl/well) with the recombinant human CD38 extracellular membrane protein (Sino Biological Inc.), incubated overnight at 4°C.
2) After washing with PBS solution and blocked with 5% milk (diluted in PBS-0.1% Tween20 solution), the plates were sealed at room temperature, followed by the addition of either different concentrations of anti-CD38 monoclonal antibodies (m29 or Daratumumab) or the non-related antibody (anti-VEGF monoclonal antibody hPV19) with a fixed concentration of the biotin-labeled Daratumumab (biotin-Daratumumab, diluted at 1:1000) or biotin-labeled m29 monoclonal antibody (biotin-m29, diluted at 1:1000) and incubated the plates at 37°C for 1.5h.
3) After eluting with PBS-T solution, the HRP-Avidin (diluted at 1:500) was added and incubated the plates at 37°C for 1 hour.
4) After eluting with PBS-T solution again, the chromogenic substrate solution OPD-3% H₂O₂ was added for staining for 10 minutes at room temperature.
5) 1M HCl solution was added to quench the reaction, then the absorption values at 492 nm wavelength were read in an enzyme-linked immunometric meter.

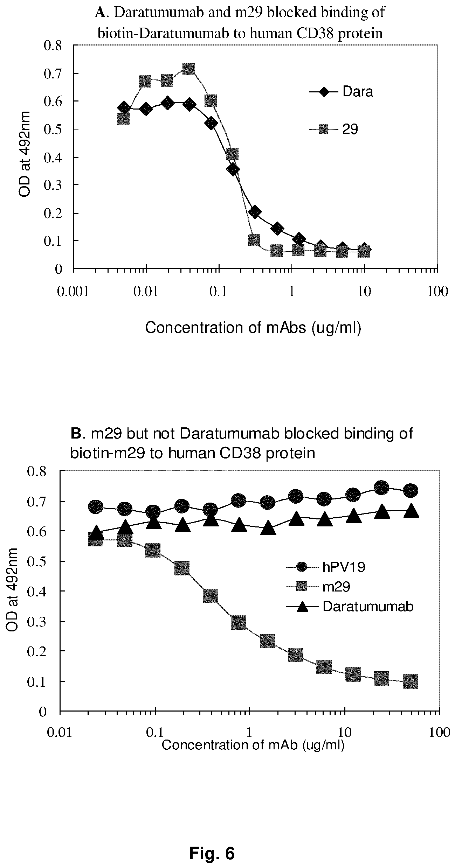

Fig. 6 is the schematic diagram showing the representative results of the competitive ELISA; wherein
Fig. 6A is the result of the competitive binding of m29 monoclonal antibody and Daratumumab with biotin-Daratumumab to CD38 in vitro. As shown in Fig. 6A, the OD value of chromogenic reaction in each well was inversely proportional to the amount of un-labeled-antibodies: namely the higher the amount of m29 monoclonal antibody or Daratumumab was added, the lower the OD value of chromogenic reaction was. The results also illustrated that similar to Daratumumab, m29 monoclonal antibody can compete with the biotin-Daratumumab in vitro for binding to CD38; furthermore, m29 monoclonal antibody also achieved almost the same competitive binding effect to CD38 as that of Daratumumab.
Fig. 6B is the in vitro competitive binding result of the m29 monoclonal antibody, Daratumumab, with the biotin-m29 monoclonal antibody to CD38. As shown in Fig. 6B, the OD value of chromogenic reaction in each well was inversely proportional to the amount of the unlabeled m29 antibodies: i.e. the higher the amount of m29 monoclonal antibody was added, the lower the OD value of chromogenic reaction was. In contrast, the amount of Daratumumab or the amount the non-related monoclonal antibody sample hPV19 had little influence on the OD value of each well. These results also illustrated that Daratumumab did not compete with the m29 monoclonal antibody for the binding to CD38 in vitro.

In summary, the competitive ELISA results showed that the CD38 binding epitope of m29 monoclonal antibody is different from Daratumumab, and m29 monoclonal antibody can competitively block the binding of Daratumumab to human CD38 protein; but in contrast, Daratumumab cannot block the binding of m29 monoclonal antibody to human CD38 protein.

### Example 7: Cloning the Gene Coding Variable Regions of Murine Monoclonal Antibody m29

Herein, the total RNA was extracted from the mouse m29 hybridoma cells, and used as a template; together with the degenerate primers, to clone and amplify the cDNA gene fragments of m29 antibody heavy chain variable region and light chain variable region, respectively, by reverse transcription-polymerase chain reaction (RT-PCR) method (Wang Y et al : Degenerated primer design to amplify the heavy chain variable region from immunoglobulin cDNA. BMC Bioinformatics. 2006; 7 Suppl (4): S9).

Wherein the cDNA gene cloning steps were as follows:
**Step 1:** The total RNA was extracted from the mouse m29 hybridoma cells with a kit (Beyotime Biotechnology, Haimen, Jiangsu)
**Step 2:** cDNA template was obtained in Eppendorf tube by RT-PCR method
Wherein, the sequence of the RT-PCR primer mKaRT for m29 antibody light chain variable region was TGT CGT TCA CTG CCA TCA AT (SEQ ID NO: 18);
The sequence of the RT-PCR primer mGaRT for m29 antibody heavy chain variable region was GCA AGG CTT ACA ACC ACA ATC (SEQ ID NO: 19);
RT-PCR reaction system was as follows :

| | |
|---|---|
| Primers | 2µl |
| RNA template | 30µl |

Incubated at 72°C for 10 minutes, then stayed on the ice for 2 minutes;
Followed by:

| | |
|---|---|
| 5×RT-PCR reaction buffer solution | 10µl |
| dNTPs | 5 µl |
| Prime Script reverse transcription-polymerase | 1.5µl |
| Distilled water | 1.5µl |
| **Total volume** | **50µl** |

The RT-PCR was reacted at 42°C for 1 hour, then increased the temperature to 75°C, after 15 minutes of inactivation, the cDNA was obtained and stored at -20°C for later use.
**Step 3:** PCR cloning and amplification of m29 antibody light chain variable region gene and heavy chain variable region gene

The following pair of primers used in cloning and amplification of m29 antibody light chain variable region gene by degenerate primers PCR method were as follows:
Forward primer mIgLF1 : GACATTGTGATGWCM CA (SEQ ID NO: 20);
Reverse primer mIgLCR440 : CTGAGGCACCTCCAGATGTT (SEQ ID NO: 21).
wherein W=A or T, M= A or C_{∘}

The following pair of primers used in cloning and amplification of m29 antibody heavy chain variable region gene by degenerate primers PCR method were as follows:
Forward primer mIgHset1 : CARCTGCARCARYCTG (SEQ ID NO: 22);
Wherein R= A or G, Y=C or T_{∘}
Reverse primer mIgHCR135 : GTGCTGGAGGGGACAGTCACT (SEQ ID NO: 23).

DNA products amplified by the PCR were analyzed by electrophoresis in 1% agarose gel. When electrophoresis was over, the separated bands were cut and subjected to DNA sequencing to obtain the nucleotide sequences of antibody light and heavy chain variable region. The nucleotide sequence of the light chain variable region was set forth in SEQ ID NO: 1. The amino acid sequence of the light chain variable region deducted from the DNA nucleotide sequence was set forth in SEQ ID NO: 2. The amino acid sequences of complementarity-determining regions (CDR) CDR1, CDR2, and CDR3 of the light chain antigen were set forth in SEQ ID NO: 3, SEQ ID NO: 4, and SEQ ID NO: 5, respectively.

The nucleotide sequence of the heavy chain variable region was set forth in SEQ ID NO: 6, and the amino acid sequence of the heavy chain variable region deducted from the DNA nucleotide sequence was set forth in SEQ ID NO: 7. The amino acid sequences of complementarity-determining regions (CDR) CDR1, CDR2, and CDR3 of the heavy chain antigen were set forth in SEQ ID NO: 8, SEQ ID NO: 9 and SEQ ID NO: 10, respectively.

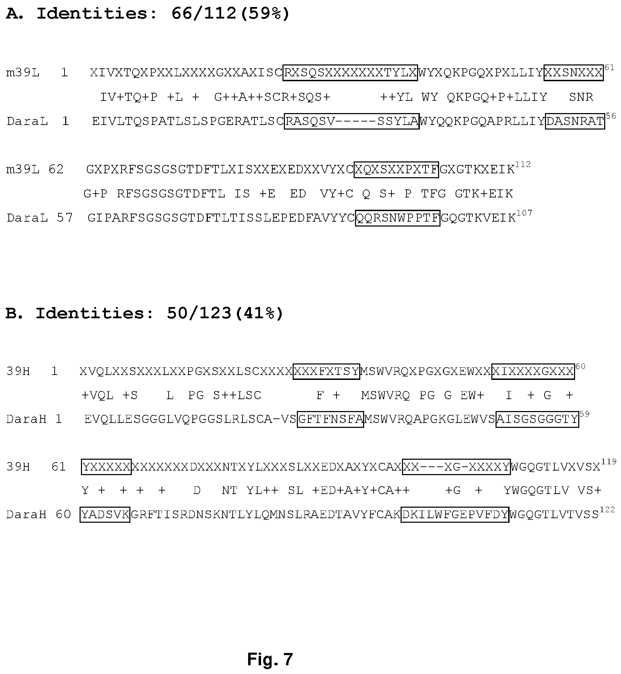

Fig. 7A is comparison analysis result of the amino acid sequences in the light chain variable regions of m29 antibody and Daratumumab; wherein different amino acid sequences of the light chain variable regions between m29 antibody and Daratumumab were marked in "X", CDR1, CDR2, and CDR3 of all kinds of antibodies light chain variable regions were marked in Box. The comparison analysis result showed that the light chain variable regions of m29 and its CDR sequences were obviously different from Daratumumab.

Fig. 7B is the comparison analysis result of the amino acid sequences in the heavy chain variable regions of m29 antibody and Daratumumab; wherein different amino acid sequences of the heavy chain variable regions between m29 antibody and Daratumumab were marked in "X", CDR1, CDR2, and CDR3 of all kinds of antibodies heavy chain variable regions were marked in Box. The comparison analysis result showed that the heavy chain variable region of m29 and its CDR sequences were obviously different from Daratumumab.

### Example 8: Construction of Human-Mouse Chimeric Antibody (ch29G) base on Murine Antibody m29

The m29 antibody light and heavy chain variable region genes obtained by the cloning and amplification in Example 7 were fused, respectively, with a human kappa light chain constant region (C-domain) and a human IgG1 heavy chain constant region fragment gene to obtain the human-mouse chimeric light chain gene (ch29L) and the human-mouse chimeric heavy chain gene (ch29H). After that, the light chain and heavy chain chimeric genes were successively cloned into the expression plasmid pQY-DHFR-Hex, then transferred into *E*. *Coli* to amplify, and obtain large amounts of the expression plasmids containing the human-mouse chimeric antibody gene.

The expression plasmids containing the human-mouse chimeric antibody gene were mixed X-tremeGENE HP DNA Transfection Reagent liposome (Roche) and transfected into CHO-DHFR⁻ cells. Two to 3 days after of cell transfection, the culture supernatants were collected and transferred to the 96-well plates pre-coated with human CD38-his protein, HRP-Goat anti-Human IgG was then added as the detection antibody (Purchased from Shanghai Xitang Biology company) to detect the binding of the chimeric antibody (ch29G) to human CD38-his protein by ELISA.

The representative ELISA results were shown in the following Table 3:

**Table 3 Detecting the binding of the cell culture supernatants from CHO transiently transfected with chimeric antibody (ch29G) gene to human CD38-his protein by ELISA**

| Supernatant dilution (folder) | 1 | 2 | 4 | 8 | 16 | 32 | 64 | 128 |
|---|---|---|---|---|---|---|---|---|
| OD492 value | 0.953 | 0.865 | 0.698 | 0.518 | 0.321 | 0.230 | 0.113 | 0.077 |

The results in Table 3 demonstrated that culture supernatants from CHO cell transfected with the human-mouse chimeric antibody ch29G gene expressing plasmid can specifically bind to human CD38 protein.

After that, the above-transfected cells were passaged to the culture dish, and the conditioned media were collected and used for screening the stable expression cell lines. After 2 to 3 weeks of culturing, the well-grown cell clones were selected and transferred into 96-well plates. After 2 to 3 days, the supernatants were collected for detecting the protein expression level in the supernatants by an ELISA. Finally, a CHO cell line with a high level expression of ch29 antibody protein was screened out. The cell line was adapted in serum-free medium (CHOM-B01, Shanghai Baian Medical Investment Co., LTD.), and after the successful adaption, the cells were amplified and supernatants were collected. After centrifugation and filtration with a 0.45 µm filter membrane, the supernatants were loaded to a Protein-A chromatography affinity column (Protein-A Sepharose Fast Flow, GE, USA), followed by isolation and purification, the buffer was changed to a glycine-Tris (pH=7.0) and the human-mouse chimeric antibody (ch29G, or ch29 for short) with a purity of over 99% was obtained.

### Example 9: Detecting and Comparing the Binding of Murine Monoclonal Antibody m29, Human-Mouse Chimeric Antibody Ch29G, and Daratumumab to Human Tumor Cell Lines Expressing CD38 Antigen by Flow Cytometer

In the present example, either the purified murine m29 monoclonal antibody, human-mouse chimeric antibody ch29G, Daratumumab or the non-related hAB21 monoclonal antibody (a humanized anti-PD-1 monoclonal antibody) were used as the primary antibodies, and FITC-Goat anti-Mouse IgG or FITC-Goat anti-human IgG was used as the detection antibody to detect the binding of samples to CD38 positive tumor cell lines by a flow cytometry method as described in Example 4.

For this purpose, human tumor cell lines that are known to express CD38 antigen such as human Burkitt B-lymphoma cell line Raji, Human myeloma cell line RPMI-8226, human T-lymphoma cell line MOLT-4, and human T-lymphoma cell line Jurkat (all purchased from Cell Center of Shanghai Institute of Life Sciences, Chinese Academy of Sciences) were incubated with either murine m29 monoclonal antibody, human-mouse chimeric antibody ch29G, Daratumumab or a non-related hAB21 monoclonal antibody at 4 °C for 1 hour, after rinsing with PBS-0.1% FCS solution, then a FITC-Goat anti-Mouse IgG (diluted at 1:200; Sigma, USA) antibody or a FITC-Goat anti-human IgG antibody (diluted at 1:200; Sigma, USA) was added and incubated the plate at 4°C for 1 hour; after rinsing with PBS-0.1% FCS solution again, the samples were loaded to BD Accuri C6Plus Flow Cytometer (Becton Dickinson, USA; Mountain View, CA) for detecting.

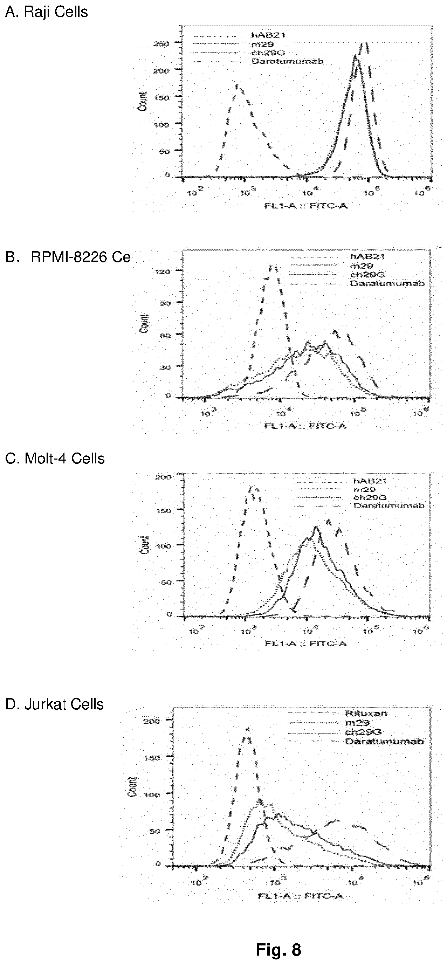

Fig. 8 shows the representative flow-cytometry analysis results; wherein
Fig. 8A is the result of human Burkitt B-lymphoma cell line Raji: compared with non-related hAB21 monoclonal antibody, mouse m29 monoclonal antibody, human-mouse chimeric antibody ch29G and Daratumumab all showed a significantly binding to human Burkitt B-lymphoma cell line; wherein the ratio of positive binding Raji cells and the binding intensity in murine m29 monoclonal antibody sample or human-mouse chimeric antibody ch29G sample were almost the same as that in Daratumumab sample.
Fig. 8B is the result of human myeloma cell line RPMI-8226: compared with non-related hAB21 monoclonal antibody, mouse m29 monoclonal antibody, human-mouse chimeric antibody ch29G and Daratumumab all showed a significantly binding to human RPMI-8226 cell line; wherein the ratio of positive binding RPMI-8226 cells or the binding intensity in murine m29 monoclonal antibody sample or in human-mouse chimeric antibody ch29G sample were also almost the same as that in Daratumumab sample.
Fig. 8C is the result of human T-lymphoma cell line MOLT-4: the ratio of positive binding MOLT-4 cells and the binding intensity in murine m29 monoclonal antibody sample or human-mouse chimeric antibody ch29G sample were slightly lower than that in Daratumumab.
Fig. 8D is the results of human T-lymphoma cell line Jurkat: the ratio of positive binding Jurkat cells and the binding intensity of murine in m29 monoclonal antibody sample or human-mouse chimeric antibody ch29G sample were lower than Daratumumab.

### Example 10: Analyzing and Comparing CDC Activities of Murine Monoclonal Antibody m29, Human-Mouse Chimeric Antibody Ch29G, and Daratumumab in Vitro

In vitro CDC activities of murine monoclonal antibody m29, human-mouse chimeric antibody ch29G, and Daratumumab were analyzed and compared using the same CDC assay as described in Example 2. In the present example, the target cells used were the classical Daudi cells; the source of complement was a 10% rabbit serum (in-house).

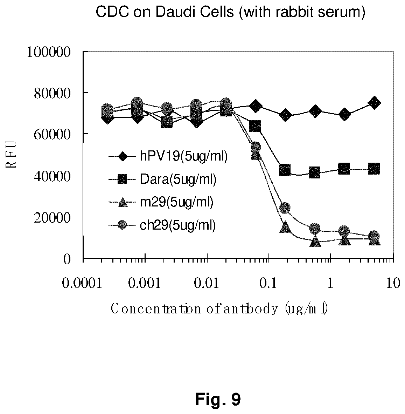

Fig. 9 shows the CDC assay results. The results showed that the CDC activities of the murine monoclonal antibody m29 or the human-mouse chimeric antibody ch29G were all stronger than Daratumumab. Their maximum CDC activity values were at more than 95%, and the EC50 values were around 30 ng/mL; in contrast, the maximum CDC activity value of Daratumumab was only at around 55%, and the EC50 value was 100 ng/mL.

### Example 11: Detecting Binding of Murine Monoclonal Antibody m29, Human-Mouse Chimeric Antibody ch29G, and Daratumumab to human CD38 with Point Mutation by Flow Cytometer

### 11.1 Construction of CHO cell lines stably expressing human CD38 gene with point mutation (CHO/hCD38-S_{274F})

In order to mutate the serine (S) at C-terminal 274 in human CD38 into phenylalanine (F), the human CD38 cDNA synthesized in Example 5.1 was used as a template to design primers for PCR amplification:
Forward Primer huCD38F- HindIII-2 :
   TTGTAAGCTTGCCGCCACCATGGCTAACTGCGAGTTCTCC (SEQ ID NO: 15) ;
Reverse primer huCD38-S274F-R1
   CTTATCGGGCCTATAGATATTTTTGCAGAAGAACTGGATGTTCCGCTTGCTGATGATGC (SEQ ID NO: 16)

The obtained DNA products were performed electrophoresis to separate the target DNA bands in 1.5% Agarose gel. Then the recycled DNA fragments were used as templates, and the following primers were used to conduct the second round of PCR to achieve the complete hCD38-S_{274F} gene.
Forward primer: huCD38F-HindIII-2:
   TTGTAAGCTTGCCGCCACCATGGCTAACTGCGAGTTCTCC (SEQ ID NO: 15);
Reverse primer: huCD38-S274F-R2-XhoI:

TGGTCTCGAGTCAGATCTCGGAGGTGCAGCTGGAGTCTTCGGGGTTCTTCACGCACTGT
AAAAACTTATCGGGCCTATAGATATT (SEQ ID NO: 17)

The hCD38-S_{274F} gene fragments were cloned into cell expression vector pQY-DHFR (in-house), then transferred into *E*. *coli,* and the positive recombinant plasmid pQY-DHFR-HCD38-S_{274F} was identified through a endonuclease digestion.

After that, the recombinant plasmid pQY-DHFR-HCD38-S274F was mixed with Fugen-6 liposome (Roche), then transfected into CHO-DHFR⁻ cells. After transfection and screening in IMDM culture medium containing FBS, the CHO cell lines stably expressing human CD38 gene with a point mutation (CHO/hCD38-S274F) were obtained.

### 11.2 Detecting the binding of murine monoclonal antibody m29, human-mouse chimeric antibody ch29G, and Daratumumab to CHO cell lines expressing wild-type CD38 or CD38 with a point mutation by flow cytometer

In the present example, the binding of the murine monoclonal antibody m29, human-mouse chimeric antibody ch29G, and Daratumumab to CHO cell lines expressing wild-type CD38 or CD38 with a point mutation was detected by flow cytometer.

The main detecting steps were as follows: CHO cells stably expressing wild-type or a point mutation of CD38 were respectively incubated with murine monoclonal antibody m29, human-mouse chimeric antibody ch29G, or Daratumumab at 4 °C for 1 hour; after washing with PBS-0.1% FCS solution, a FITC-Goat anti-Mouse IgG antibody or a FITC-Goat anti-Human IgG antibody was added, incubated at 4 °C for 1 hour, then washing with PBS-0.1% FCS solution again, the samples were loaded into Accuri C6 Plus Flow Cytometer (BD, USA) for testing.

Fig. 10 shows the schematic diagrams of the representative flow cytometry results, wherein
Fig. 10A is the result in CHO cell lines expressing wild-type human CD38: it showed that the murine monoclonal antibody m29 sample, human-mouse chimeric antibody ch29G sample all retained the binding to CHO cells expressing the wild-type human CD38 (CHO/hCD38-wild-type) and the binding intensity seen in these two samples is similar to that of Daratumumab.
Fig. 10B is the result in CHO cells expressing CD38 with a point mutation (CHO/hCD38-S_{274F}): it showed that the murine monoclonal antibody m29 sample, human-mouse chimeric antibody ch29G sample also retained the binding to the CHO cells expressing the CD38 with a point mutation (CHO/hCD38-wild-type); in contrast, Daratumumab did not bind to CHO cells expressing CD38 with this point mutation.

### Example 12: Construction of CHO Cell Lines (CHO/mkCD38) Stably Expressing Cynomolgus Macaque CD38 and Detecting the Binding of Murine Monoclonal Antibody m29, Human-Mouse Chimeric Antibody ch29G, and Daratumumab to Them

### 12.1 Construction of CHO cell lines (CHO/mkCD38) stably expressing cynomolgus macaque CD38

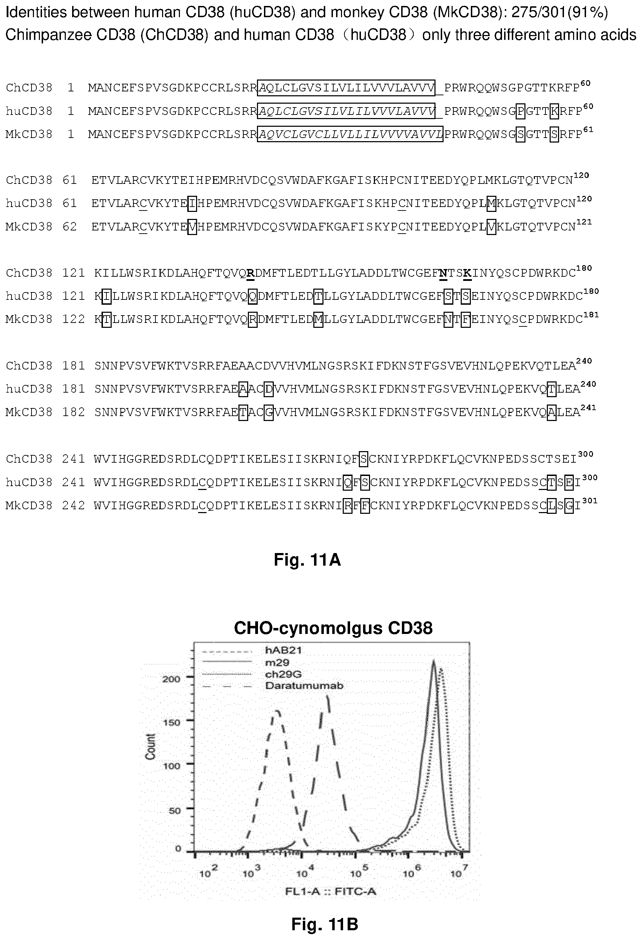

A comparison analysis result of the amino acid sequences of human, cynomolgus macaque (Cynomolgus monkey) and chimpanzee (Pan troglodytes) CD38 protein was shown in Fig. 11A. As shown in Fig. 11A, the amino acid sequence of chimpanzee CD38 protein (ChiCD38) was almost the same with human CD38 protein (huCD38); while the amino acid sequence of cynomolgus macaque CD38 (mkCD38) protein is 91% identity to that of human CD38 protein, with 16 amino acid differences.

According to the gene sequence of cynomolgus macaque CD38 published in Genbank (Gene ID: 102126394), the responding cDNA fragments coding the cynomolgus macaque CD38 were synthesized by Suzhou Genewiz Biological Technology Co. LTD and then cloned into the expression plasmid pQY-DHFR (in-house), transferred into *E*. *coli,* after that, the positive recombinant plasmid pQY-DHFR-mkCD38 was identified through endonuclease digestion.

After that, the recombinant plasmid pQY-DHFR-mkCD38 was mixed with Fugen-6 liposome (Roche), then co-transfected into DHFR gene deficiency CHO cells (CHO-dhfr). After transfection, screened by IMDM culture medium containing FBS, the cell lines expressing cynomolgus macaque CD38 protein (CHO-cynomolgus CD38) were obtained.

### 12.2 Detecting binding of murine monoclonal antibody m29, human-mouse chimeric antibody ch29G, and Daratumumab to CHO cell lines stably expressing cynomolgus macaque CD38 (CHO-cynomolgus CD38) by flow cytometer

The binding of murine monoclonal antibody m29, human-mouse chimeric antibody ch29G, and Daratumumab to CHO cell lines stably expressing cynomolgus macaque CD38 (CHO-cynomolgus CD38) was detected by the flow cytometry method described in section 11.2 of Example 11.

Fig. 11B is a schematic diagram of the representative flow cytometry results, and the result showed that the human-mouse chimeric antibody ch29G and the mouse monoclonal antibody m29 retained the same high affinity to CHO cells (CHO-cynomolgus CD38) stably expressing cynomolgus macaque CD38; instead, the binding intensity of Daratumumab to CHO cells expressing cynomolgus macaque CD38 was reduced by more than 90%.

### Example 13: Detecting the Binding of Human-Mouse Chimeric Antibody ch29G or Daratumumab to Human and Cynomolgus Macaque CD38 Protein by ELISA

In the present example, the binding of the human-mouse chimeric antibody ch29G and Daratumumab to the recombinant human CD38 protein or the recombinant cynomolgus macaque CD38 protein was detected by a direct ELISA.

For this purpose, 96-well cell culture plates were coated with the recombinant human CD38-his protein (Sino Biological Inc.) or the recombinant cynomolgus macaque CD38-his protein (Sino Biological Inc.) (1 µg/mL, 50 µl/well, 4°C overnight), and blocked with 5% milk. After blocking, the human-mouse chimeric antibody ch29G sample, Daratumumab, or the negative control antibody Rituximab (anti-human CD20 monoclonal antibody) were diluted to 1 µg/mL with diluent (PBST-5% milk), then loaded to 96-well plates pre-coated with CD38 protein, 12 wells were diluted in 2-fold serial dilution, incubated at room temperature for 1 hour; after thoroughly washing with PBS-0.1% Tween20 solution, HRP enzyme labeled Goat-anti-human-IgG was added as the detection antibody (purchased from Shanghai Xitang Biology company, diluted at 1:1000) and incubated the plates at room temperature for 1 hour; after thoroughly washing with PBS-0.1% Tween20 solution again, the substrate solution OPD-0.1% H₂O₂ was added for a color development at room temperature for about 10-15 minutes, then 1M HCl solution was added to quench the reaction, and the OD values at 492 nm wavelength were recorded in a multimode reader (PerkinElmer Victor X3).

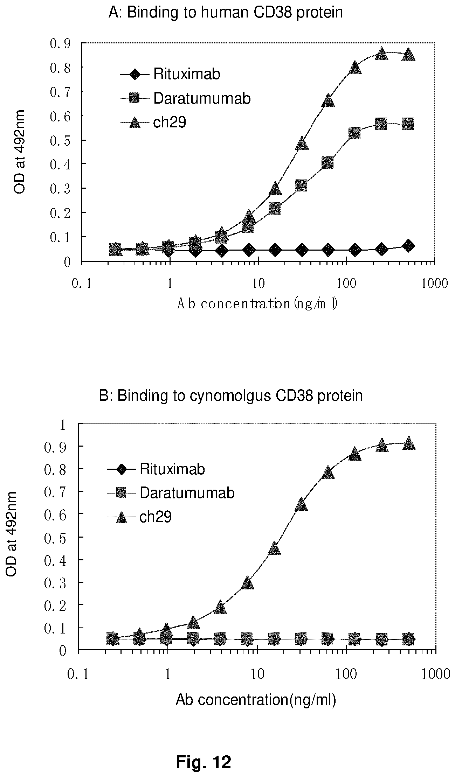

Fig. 12 is the representative ELISA result. As shown in Fig. 12, while both the human-mouse chimeric antibody ch29G and Daratumumab had the same high affinity binding to human CD38 protein (Fig. 12A), only the human-mouse chimeric antibody ch29G also had a high affinity binding to cynomolgus macaque CD38 protein, and, Daratumumab did not show any significant binding to cynomolgus macaque CD38 protein (Fig. 12B).

### Example 14: Comparing and Analyzing the CDC Activities of Human-Mouse Chimeric Antibody ch29G and Daratumumab in Vitro

### 14.1 Materials and method

In the present example, the CDC activities of the human-mouse chimeric antibody ch29G to several target cells including Daudi, Raji, MOLT-4, and Jurkat, etc. were assayed by the same CDC method as described in Example 2, and the results were compared with that of Daratumumab. Wherein the complement used in the assay was the 10 % huama serum from a healthy donor (in-house-made), the positive control sample for the assay was Rituximab (a human-mouse chimeric anti-CD20 monoclonal antibody), and the negative control sample was the hPV19 monoclonal antibody (a humanized anti-VEGF monoclonal antibody).

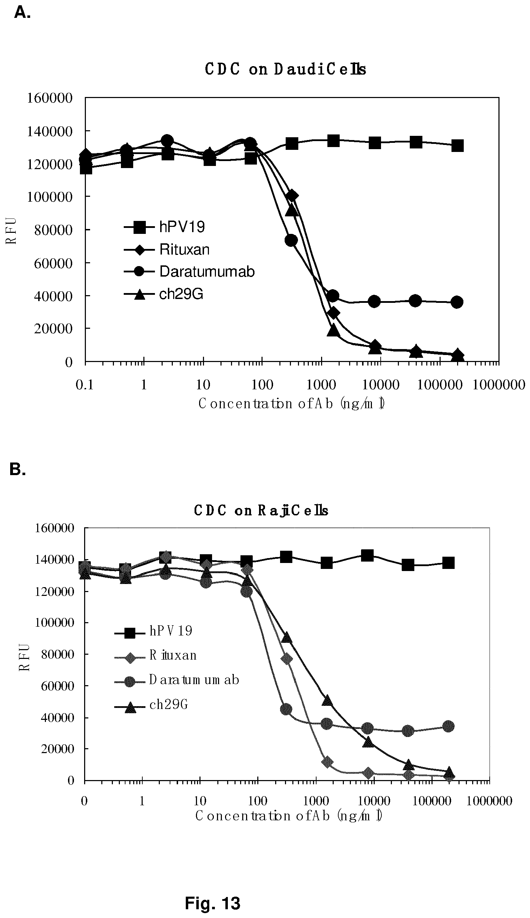

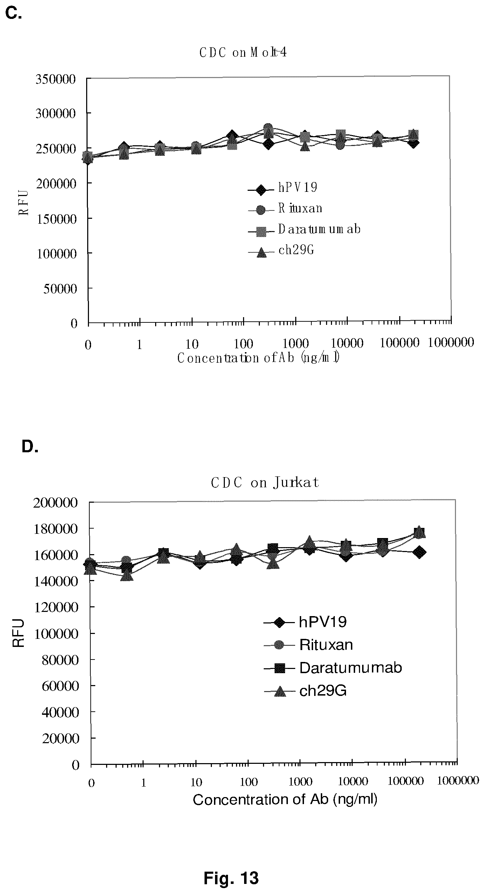

Fig. 13 shows the CDC assay results, wherein
Fig. 13A shows the CDC assay results with Daudi as the targeting cell, and the results showed that the human-mouse chimeric antibody ch29G had a similar CDC activity as that of the positive control sample Rituximab; Furthermore, the CDC activity in each of these two samples was stronger than that of Daratumumab.
Fig. 13B shows the CDC assay results with Raji as the targeting cell, and the results showed that the human-mouse chimeric antibody ch29G also had a similar CDC activity as that of the positive control sample Rituximab; and furthermore, the CDC activity of Daratumumab was also similar to that of human-mouse chimeric antibody ch29G.
Fig. 13C shows the CDC assay results with MOLT-4 as the targeting cell, and the results showed that the human-mouse chimeric antibody ch29G, Daratumumab, and Rituximab all had no significant CDC activities.
Fig. 13D shows the CDC assay results with Jurkat as the targeting cell, and the results showed that the human-mouse chimeric antibody ch29G, Daratumumab, and Rituximab all had no significant CDC activities.

### Example 15: Humanization of Murine Antibody m29 by Genetic Engineering

As the preliminary data from ELISA, CDC assay and, etc. showing the human-mouse chimeric antibody ch29G has a high affinity binding to human CD38 and CDC activity to human CD38 positive cells, a series of genetic engineering and cloning methods such as PCR were used to transplant the antigen complementarity-determining regions (CDRs) gene fragments from the chimeric antibody light chain to the corresponding human Kappa light chain variable framework regions (FR), thus a humanized antibody light chain was obtained. This light chain was then combined with the chimeric heavy chain, therefore, HH29G antibody with the humanized light chain was achieved.

### 15.1 Humanization of murine m29 antibody light chain by genetic engineering

Upon amino acid sequence analysis, the expression product of the first V germline Gene of human immunoglobulin Kappa light chain (IgKV2D-29, NCBI Gene ID: 28882) was determined to have the highest identity with the light chain variable region of murine m29 antibody. Accordingly, the light chain framework region (FR) of m29 antibody was replaced with the homologous sequence of human IGKV2D-29, then the replaced variable region gene was jointed with the sequence coding the light chain constant region of human immunoglobulin IgG-Kappa, and finally, the humanized light chain coding gene (H29-L) was successfully obtained. The amino acid sequence of the light chain variable region of the humanized m29 antibody was shown in SEQ ID NO: 11, and its nucleotide sequence was shown in SEQ ID NO: 13.

### 15.2 Humanization of murine m29 antibody heavy chain by genetic engineering

Upon amino acid sequences analysis, the expression product of the first V germline Gene of human immunoglobulin Kappa heavy chain (IGHV1-69, NCBI Gene ID:28461) was determined to have the highest identity with the heavy chain variable region of m29. Accordingly, the heavy chain framework region (FR) of m29 was replaced with the homologous sequence of human IGHV1-69, then the replaced variable region gene was jointed with the coding sequence of the heavy chain constant region of human immunoglobulin IgG, and finally, the humanized heavy chain coding gene (h29-h) was successfully obtained. The amino acid sequence of the heavy chain variable region of the humanized m29 antibody was shown in SEQ ID NO: 12, and its nucleotide sequence was shown in SEQ ID NO: 14.

### Example 16: Engineering Construction of CHO Cell Lines Stably and High Efficacy Expressing Humanized or Half Humanized HH29 Monoclonal Antibody (HH29) and Isolation and Purification of Antibody Protein.

The human-mouse chimeric heavy chain gene (ch29H) and the humanized light chain gene (HH29L ) were cloned step-by-step to the expression vector pQY-Dhfr-Hex, then transferred into *E*. *coli,* after amplification and purification, the expression plasmids containing humanized or half humanized monoclonal antibody (HH29) were obtained. The expressing recombinant plasmid with HH29 or ch29 was transiently transfected into CHO cells. About 24 hours after transfection, cell supernatants were collected from the wells, and used for detecting the binding to human CD38 protein antigen by a direct ELISA with CD38-his protein as the coated antigen, HRP-Goat anti-Human IgG as the detection antibody (purchased from Shanghai Xitang Biological Company), OPD used as the chromogenic substrate.

Table 4 shows the representative ELISA results.

**Table 4 Binding activity of transiently transfected cell culture supernatant to human CD38 protein**

| Supernatant Dilution Folder | OD Values | |
|---|---|---|
| | ch29 | HH29 |
| 2 | 0.989 | 1.177 |
| 4 | 0.945 | 1.163 |
| 8 | 0.789 | 1.105 |
| 16 | 0.603 | 0.811 |
| 32 | 0.431 | 0.508 |
| 64 | 0.313 | 0.325 |
| 128 | 0.212 | 0.178 |
| 256 | 0.121 | 0.110 |

As shown in Table 4, like that in human-mouse chimeric monoclonal antibody ch29G, the half humanized monoclonal antibody HH29 (light chain humanized) retained the binding activity to human CD38 protein.

The above-transfected cells were adapted to the serum-free medium suspension culture and subjected to a clonal screening, and several engineering CHO cell lines with a stable and efficient expression of the half humanized monoclonal antibody HH29 (light chain humanized) were obtained.

Next, one engineering cell line was chosen to amplify in a serum-free medium, and the culture supernatants were collected. After centrifugation and filtration with a 0.45 µm filtration membrane, and multiple purification steps including a Protein-A affinity chromatography column (protein A-Sepharose Fast Flow, GE, USA, ion exchange column separation, virus removal/inactivation, and filtration/sterilization (0.22 µm filtration membrane), the HH29 antibody with a high purity (protein purity > 99%) was obtained. The purified HH29 monoclonal antibody was dissolved in a glycine-Tris buffer (pH=7.0) (1-10 mg/mL) and stored at low temperature (about 4 °C).

### Example17 : Detecting Anti-Tumor Efficacy of Mouse Monoclonal Antibody m29 in Vivo

In the present example, the anti-tumor efficacy of the murine monoclonal antibody m29 was tested in vivo by subcutaneous inoculation of human B-lymphoma Raji tumors in nude mice, and the human-mouse chimeric anti-CD20 monoclonal antibody Rituximab used as a positive control drug. Human B-lymphoma Raji cells were first inoculated subcutaneously in nude mice; after tumor established, the animals were divided into different groups and administered with treatment agents. The tumor growth was observed and recorded.

Briefly, 1×10⁷ human B-lymphoma Raji cells (from Typical Culture Preservation Commission Cell Bank, Chinese Academy of Sciences) were inoculated in nude mice (purchased from Nanjing University Animal Center), and when the inoculated tumor volume grew to about the size of a soybean (about 100 mm³, approximately 6-7 days after tumor cells inoculation), the animals were randomly divided into the following three groups:
Group A: normal saline negative control group (n=2, equal volume normal saline);
Group B: positive control drug Rituximab treatment group (n=4, the dosage was 10 mg/kg body weight);
Group C: m29 monoclonal antibody treatment group (n=4, the dosage was 10 mg/kg body weight).

On the same day (i.e., 6-7 days after the tumor inoculation), the animals were administered of the drugs by an intraperitoneal injection (i.p.), then i.p injections were repeated twice a week (every 3-4 days) consecutively for six times (3 weeks). During the administering period, the general clinical symptoms were observed every day, and the long diameter (mm) and short diameter (mm) of the tumors and the animal weight were measured every 3-4 days.

The tumor volume calculation formula is volume (mm³) = long diameter (mm)x short diameter (mm)x short diameter (mm)x0.5. If the tumor volume exceeded 3,000 mm³ at the time of the measurement, the testing animals would be euthanized.

### Testing Results:

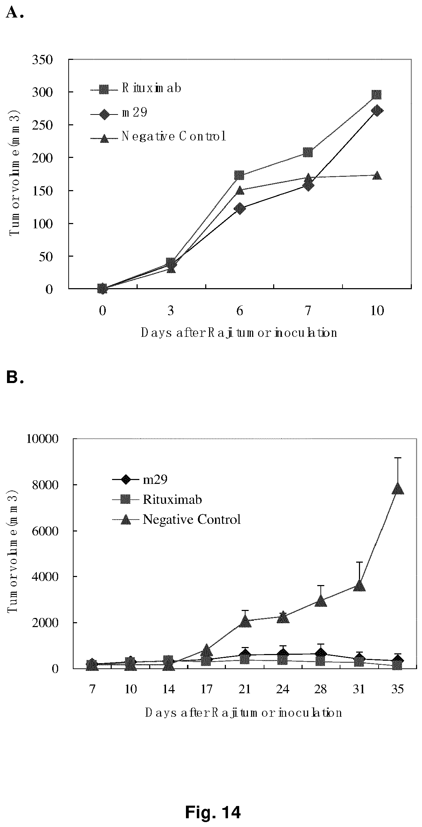

Fig. 14 shows the average tumor growth volume trend in animals from each experimental group.

Fig. 14A is a schematic diagram showing the average tumor growth volume during the first 10 days after tumor inoculation, and the results showed that there is no significant difference between each group.

Fig. 14B is a schematic diagram showing the average tumor growth volume during the later stage after tumor inoculation. The results showed that, compared with the normal saline negative control group, the tumor growth in the m29 monoclonal antibody treatment group or in the positive control drug Rituximab treatment group was significantly reduced or even completely disappeared.

### Example 18: Testing Anti-Tumor Efficacy of Human-Mouse Chimeric Antibody ch29G in Vivo

In the present example, the anti-tumor efficacy of the human-mouse chimeric antibody ch29G was tested in vivo by a subcutaneous inoculation of human B-lymphoma Raji tumor cells in nude mice with a same method as described in Example 17, and Daratumumab was used as the positive control drug. Human B-lymphoma Raji cells were first inoculated subcutaneously in nude mice. After the tumors were formed in testing animals, the animals were divided into different groups and administered with treatment agents, and the tumor growth was observed and recorded.

Breifly, 1×10⁷ human B-lymphoma Raji cells were inoculated in nude mice (Purchased from Nanjing University Animal Center), when the inoculated tumor volume grew to about the size of a soybean (about 100 mm³, approximately 6-7 days after tumor cells inoculated), the animals were randomly divided into the following three groups:
Group A: normal saline negative control group (n=2, equal volume normal saline);
Group B: positive control drug Daratumumab group (n=10, the dosage was 5 mg/kg body weight);
Group C: ch29G monoclonal antibody treatment group (n=10, the dosage was 2.5 mg/kg body weight).

On the same day (i.e., 6-7 days after tumor inoculation), the animals were administered of the treatment agents by intraperitoneal injection (i.p.), then the ip. injections were repeated twice a week (every 3-4 days) and consecutively for six times (3 weeks). During the administering period, the general clinical symptoms of the animals were observed every day, and the long diameter (mm) and short diameter (mm) of the tumors, and the animal weight were measured every 3-4 days.

The tumor volume calculation formula is volume (mm³) = long diameter (mm)x short diameter (mm)x short diameter (mm)x0.5. If the tumor volume exceeded 3,000 mm³ at the time of the measurement, the tested animals would be euthanized.

### Testing Results:

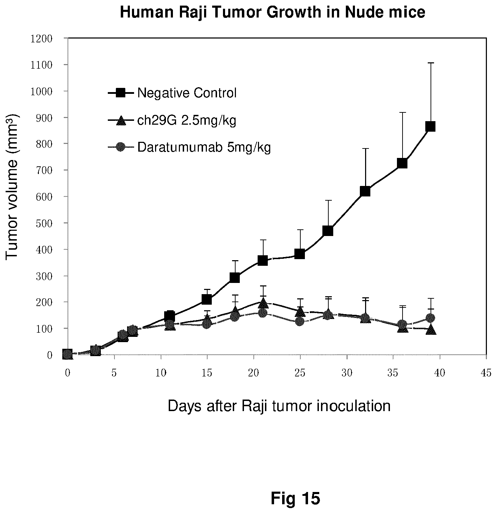

Fig. 15 shows the average tumor growth volume trend in animals from each experimental group, and the results showed that, compared with the normal saline negative control group, the tumor growth in the ch29G monoclonal antibody treatment group or in the positive control drug Daratumumab treatment group was significantly inhibited, the therapeutic efficacy of ch29G monoclonal antibody at a dose of 2.5 mg/kg body weight was similar to Daratumumab at a dose of 5 mg/kg body weight.

### References

1. Jackson DG and Bell JI. Isolation of a cDNA encoding the human CD38 (T10) molecule, a cell surface glycoprotein with an unusual discontinuous pattern of expression during lymphocyte differentiation. J. Immunol. 1990, 144:2811-2815.
2. Harada N, Santos-Argumedo L, Chang R, Grimaldi JC, Lund FE, Brannan CI, Copeland NG, Jenkins NA, Heath AW, Parkhouse RM, et al. Expression cloning of a cDNA encoding a novel murine B cell activation marker. homology to human CD38. J Immunol. 1993, 151:3111-8.
3. Ferrero E, Orciani M, Vacca P, Ortolan E, Crovella S, Titti F, Saccucci F, Malavasi F. Characterization and phylogenetic epitope mapping of CD38 ADPR cyclase in the cynomolgus macaque. BMC Immunol. 2004, 5:21.
4. States DJ, Walseth TF and Lee HC. Similarities in amino acid sequences of Aplysia ADP-ribosyl yclase and human lymphocyte antigen CD38. Trends Biochem. Sci. 1992, 17 : 495.
5. Howard M, Grimaldi JC, Bazan JF, Lund FE, Santos-Argumedo L, Parkhouse RM, Walseth TF, Lee HC. Formation and hydrolysis of cyclic ADP-ribose catalyzed by lymphocyte antigen CD38. Science. 1993, 262:105.
6. Summerhill RJ, Jackson DG, Galione A. Human lymphocyte antigen CD38 catalyzes the production of cyclic ADP-ribose. FEBS Lett. 1993, 335:231-233.
7. Sridhar Prasad, Duncan E. McRee, Enrico A. Stura, David G. Levitt, Hon Cheung Lee & C. David Stout. Crystal structure of Aplysia ADP ribosyl cyclase, a homologue of the bifunctional ectozyme CD38. Nature Structural Biology 1996, 3: 957-964.
8. Mehta K, Shahid U and Malavasi F (Review): Human CD38, a cell-surface protein with multiple function. FASEB J. 1996, 10; 1408-1417.
9. George Shubinsky, Michael Schlesinger (Review): The CD38 Lymphocyte Differentiation Marker: New Insight into Its Ectoenzymatic Activity and Its Role as a Signal Transducer. Immunity 1997, 7:315-324.
10. Stevenson FK, Bell AJ, Cusack R, Hamblin TJ, Slade CJ, Spellerberg MB, Stevenson GT. Preliminary studies for an immunotherapeutic approach to the treatment of human myeloma using chimeric anti-CD38 antibody. Blood. 1991, 77:1071-1079.
11. Goldmacher VS, Bourret LA, Levine BA, Rasmussen RA, Pourshadi M, Lambert JM, Anderson KC. Anti-CD38-blocked ricin: an immunotoxin for the treatment of multiple myeloma. Blood. 1994, 84:3017-25.
12. Ellis JH, Barber KA, Tutt A, Hale C, Lewis AP, Glennie MJ, Stevenson GT, Crowe JS. Engineered anti-CD38 monoclonal antibodies for immunotherapy of multiple myeloma. J Immunol. 1995, 155:925-937.
13. de Weers M, Tai YT, van d V, Bakker JM, Vink T, Jacobs DC, et al. Daratumumab, a novel therapeutic human CD38 monoclonal antibody, induces killing of multiple myeloma and other hematological tumors. J Immunol. 2011, 186:1840-1848.
14. Lokhorst HM, Plesner T, Laubach JP, Nahi H, Gimsing P, Hansson M, et al. Targeting CD38 with Daratumumab Monotherapy in Multiple Myeloma. N Engl J Med. 2015, 373:1207-1219.
15. Lonial S, Weiss BM, Usmani SZ, Singhal S, Chari A, Bahlis NJ, et al. Daratumumab monotherapy in patients with treatment-refractory multiple myeloma (SIRIUS): an open-label, randomised, phase 2 trial. Lancet. 2016, 387:1551-1560.
16. Nijhof IS, Groen RW, Noort WA, van KB, de Jong-Korlaar R, Bakker J, et al. Preclinical evidence for the therapeutic potential of CD38-targeted immuno-chemotherapy in multiple myeloma matients refractory to lenalidomide and bortezomib. Clin Cancer Res. 2015, 21:2802-10.
17. Plesner T, Arkenau HT, Gimsing P, Krejcik Lemech C, Minnema MC, et al. Phase 1/2 study of daratumumab, lenalidomide, and dexamethasone for relapsed multiple myeloma. Blood. 2016, 128:1821-28.
18. Palumbo A, Chanan-Khan A, Weisel K, Nooka AK, Masszi T, Beksac M, et al (CASTOR Investigators) Daratumumab, bortezomib, and dexamethasone for multiple myeloma. N Engl J Med. 2016, 375:754-66.
19. Dimopoulos MA, Oriol A, Nahi H, San-Miguel J, Bahlis NJ, Usmani SZ, et al (POLLUX Investigators). Daratumumab, lenalidomide, and dexamethasone for multiple myeloma. N Engl J Med. 2016, 375:1319-1331.
20. Ajai Chari, et al.(EQUULEUS; MMY1001 Investigators): Daratumumab plus pomalidomide and dexamethasone in relapsed and/or refractory multiple myeloma. Blood. 2017,130: 974-981.
21. Mateos M-V et al (ALCYONE Trail Investigators). Daratumumab pls bortezomib, melphalan and prednisone for untreated myeloma. N Engl J Med. 2018, 378:518-528.

## Claims

1. A monoclonal antibody or a derivative thereof, wherein the monoclonal antibody or the derivative thereof specifically binds to human and cynomolgus CD38 antigens, and comprises a first variable region and a second variable region,
wherein the first variable region is an antibody light chain variable region comprising complementarity-determining regions CDR1, CDR2, and CDR3 having amino acid sequences as set forth in SEQ ID NO: 3, SEQ ID NO: 4 and SEQ ID NO: 5, respectively; and
wherein the second variable region is an antibody heavy chain variable region comprising complementarity-determining regions CDR1, CDR2, and CDR3 having amino acid sequences as set forth in SEQ ID NO: 8, SEQ ID NO: 9 and SEQ ID NO: 10, respectively.

2. The monoclonal antibody or the derivative thereof according to claim 1, wherein the first variable region is an antibody light chain variable region having an amino acid sequence as set forth in SEQ ID NO: 2; and the second variable region is an antibody heavy chain variable region having an amino acid sequence as set forth in SEQ ID NO: 7.

3. The monoclonal antibody or the derivative thereof according to claim 1, wherein the first variable region is an antibody light chain variable region having an amino acid sequence as set forth in SEQ ID NO: 11; and wherein the second variable region is an antibody heavy chain variable region having an amino acid sequence as set forth in SEQ ID NO: 12 or SEQ ID NO: 7.

4. The monoclonal antibody or the derivative thereof according to any one of claims 1-3, comprising the antibody light chain variable region, a human antibody light chain constant region, the antibody heavy chain variable region, and a hinge region of a human antibody heavy chain constant region, CH1 region, CH2 region, and CH3 region.

5. The monoclonal antibody or the derivative thereof according to claim 4, wherein the human antibody light chain constant region is a kappa chain or a lambda chain of a human antibody, the human antibody heavy chain constant region is one selected from the group consisting of human IgG1, IgG2, IgG3, and IgG4 isotypes.

6. A DNA molecule or gene coding the monoclonal antibody or the derivative thereof according to claim 3, wherein a nucleotide sequence of the antibody light chain variable region is as set forth in SEQ ID NO: 1 or SEQ ID NO: 13, and a nucleotide sequence of the antibody heavy chain variable region is as set forth in SEQ ID NO: 6 or SEQ ID NO: 14.

7. An expression vector, comprising a DNA sequence of the DNA molecule of claim 6 and an expression regulatory sequence operably linked to the DNA sequence.

8. A recombinant host cell, wherein the recombinant host cell is transfected with the expression vector of claim 7.

9. The recombinant host cell according to claim 8 or a progeny cell thereof, wherein the recombinant host cell or the progeny cell thereof expresses the monoclonal antibody or the derivative thereof of claim 1 or claim 5.

10. A pharmaceutical compound or a pharmaceutical composition, comprising the monoclonal antibody or the derivative thereof of claim 1 or claim 5, and a pharmaceutically accepted carrier for use in treatment of tumors.

11. The pharmaceutical compound or a pharmaceutical composition, comprising the monoclonal antibody or the derivative thereof of claim 1 or claim 5, and a pharmaceutically accepted carrier for use according to claim 10 wherein the tumors are CD38-positive tumors.

12. The pharmaceutical compound or a pharmaceutical composition, comprising the monoclonal antibody or the derivative thereof of claim 1 or claim 5, and a pharmaceutically accepted carrier for use according to claim 11, wherein the CD38-positive tumors are human myeloma or human lymphoma.

13. A method for preparing the monoclonal antibody or the derivative thereof of claim 1 or claim 5, wherein the method comprises the following steps:
a) providing an expression vector, wherein the expression vector comprises a DNA molecular sequence encoding for the monoclonal antibody, or a derivative thereof, of claim 1 or claim 5 and an expression regulatory sequence operably linked to the DNA molecular sequence;
b) transfecting a host cell with the expression vector of step a);
c) culturing the host cell from step b) under conditions suitable for an expression of the monoclonal antibody or the derivative thereof; and
d) isolating, purifying, and collecting the monoclonal antibody or the derivative thereof from a host cell culture medium by an affinity chromatography.

## Patentansprüche

1. Monoklonaler Antikörper oder ein Derivat, worin der monoklonale Antikörper oder das Derivat spezifisch an menschliche und Cynomolgus-CD38-Antigene bindet und eine erste und zweite variable Region umfasst,
worin die erste variable Region eine variable Region der leichten Kette von Antikörper ist, die komplementär bestimmende Regionen CDR1, CDR2 und CDR3 mit Aminosäuresequenzen wie in SEQ ID NR: 3, SEQ ID NR: 4 bzw. SEQ ID NR: 5 dargelegt umfasst; und
worin die zweite variable Region eine variable Region der schweren Kette von Antikörper ist, die komplementär bestimmende Regionen CDR1, CDR2 und CDR3 mit Aminosäuresequenzen gemäß SEQ ID NR: 8, SEQ ID NR: 9 bzw. SEQ ID NR: 10 umfasst.

2. Monoklonaler Antikörper oder das Derivat nach Anspruch 1, worin die erste variable Region eine variable Region der leichten Kette von Antikörper mit einer Aminosäuresequenz wie in SEQ ID NR: 2 dargelegt ist; und die zweite variable Region eine variable Region der schweren Kette von Antikörper mit einer Aminosäuresequenz wie in SEQ ID NR: 7 dargelegt ist.

3. Monoklonaler Antikörper oder das Derivat nach Anspruch 1, worin die erste variable Region eine variable Region der leichten Kette von. Antikörper mit einer Aminosäuresequenz wie in SEQ ID NR: 11 dargelegt ist; und worin die zweite variable Region eine variable Region der schweren Kette von Antikörper mit einer Aminosäuresequenz wie in SEQ ID NR: 12 oder SEQ ID NR: 7 dargelegt ist.

4. Monoklonaler Antikörper oder das Derivat nach einem der Ansprüche 1 bis 3, umfassend die variable Region der leichten Kette von Antikörper, eine konstante Region der leichten Kette von menschlichem Antikörper, die variable Region der schweren Kette von Antikörper und eine Scharnierregion einer konstanten Region der schweren Kette von menschlichen Antikörper, CHI-Region, CH2-Region und CH3-Region.

5. Monoklonaler Antikörper oder das Derivat nach Anspruch 4, worin die konstante Region der leichten Kette von menschlichem Antikörper eine kappa-Kette oder eine lambda-Kette von menschlichem Antikörpers ist, die konstante Region der schweren Kette von menschlichem Antikörper eine ist, die aus der Gruppe ausgewählt ist, die aus den menschlichen Isotypen IgG1, IgG2, IgG3 und IgG4 besteht.

6. DNA-Molekül oder Gen, das den monoklonalen Antikörper oder das Derivat nach Anspruch 3 kodiert, worin eine Nukleotidsequenz der variablen Region der leichten Kette von Antikörper wie in SEQ ID NR: 1 oder SEQ ID NR: 13 dargelegt ist, und eine Nukleotidsequenz der variablen Region der schweren Kette von Antikörper wie in SEQ ID NR: 6 oder SEQ ID NR: 14 dargelegt ist.

7. Expressionsvektor, umfassend eine DNA-Sequenz des DNA-Moleküls nach Anspruch 6 und eine funktionsfähig mit der DNA-Sequenz verbundene Expressionsregulationssequenz.

8. Rekombinante Wirtszelle, worin die rekombinante Wirtszelle mit dem Expressionsvektor nach Anspruch 7 transfiziert ist

9. Rekombinante Wirtszelle nach Anspruch 8 oder eine Nachkommenschaft davon, worin die rekombinante Wirtszelle oder die Nachkommenschaft davon den monoklonalen Antikörper oder das Derivat nach Anspruch 1 oder Anspruch 5 exprimiert.

10. Pharmazeutische Verbindung oder pharmazeutische Zusammensetzung, umfassend den monoklonalen Antikörper oder das Derivat nach Anspruch 1 oder 5 und einen pharmazeutisch akzeptierten Träger zur Verwendung
bei der Behandlung
von Tumoren.

11. Pharmazeutische Verbindung oder pharmazeutische Zusammensetzung, umfassend den monoklonalen Antikörper oder das Derivat nach Anspruch 1 oder 5 und einen pharmazeutisch akzeptierten Träger
zur Verwendung
nach Anspruch 10, wobei die Tumore CD38-positive Tumore sind.

12. Pharmazeutische Verbindung oder pharmazeutische Zusammensetzung, umfassend den monoklonalen Antikörper oder das Derivat nach Anspruch 1 oder 5 und einen pharmazeutisch akzeptierten Träger
zur Verwendung
nach Anspruch 11, worin die CD38-positiven Tumore ein menschliches Myelom oder ein menschliches Lymphom sind.

13. Verfahren zur Herstellung des monoklonalen Antikörpers oder des Derivats nach Anspruch 1 oder 5, worin das Verfahren die folgenden Schritte umfasst:
a) Bereitstellen eines Expressionsvektors, worin der Expressionsvektor eine DNA-Molekularsequenz, die für den monoklonalen Antikörper oder ein Derivat davon codiert, umfasst,
nach Anspruch 1 oder 5 kodiert, und eine Expressionsregulationssequenz, die funktionsfähig mit der molekularen DNA-Sequenz verbunden ist;
b) Transfektion einer Wirtszelle mit dem Expressionsvektor aus Schritt a);
c) Züchten der Wirtszelle aus Schritt b) unter Bedingungen, die für eine Expression des monoklonalen Antikörpers oder des Derivats geeignet sind; und
d) Isolieren, Reinigen und Sammeln des monoklonalen Antikörpers oder des Derivats davon aus einem Wirtszellkulturmedium durch Affinitätschromatographie.

## Revendications

1. Un anticorps monoclonal ou un dérivé de celui - ci, **caractérisé en ce que** l'anticorps monoclonal ou le dérivé se lie spécifiquement aux antigènes CD38 humains, et crabiers et comprend une première région variable et une deuxième région variable,
Dans laquelle la première région variable est une région variable de la chaîne légère de l'anticorps comprenant des régions déterminantes complémentaires CDR1, CDR2 et CDR3 ayant des séquences d'acides aminés telles que décrites dans SEQ ID No: 3, SEQ ID No: 4 et SEQ ID No: 5, respectivement; et
Dans laquelle la deuxième région variable est une région variable de la chaîne lourde de l'anticorps comprenant des régions déterminantes complémentaires CDR1, CDR2 et CDR3 ayant des séquences d'acides aminés telles que décrites dans SEQ ID No: 8, SEQ ID No: 9 et SEQ ID No: 10, respectivement.

2. Un anticorps monoclonal ou ses dérivés selon la revendication 1, **caractérisé en ce que** la première région variable est une région variable de la chaîne légère de l'anticorps ayant la séquence d'acides aminés SEQ ID No: 2, et ladite deuxième région variable est une région variable de la chaîne lourde de l'anticorps ayant ladite séquence d'acides aminés dans SEQ ID No: 7.

3. Un anticorps monoclonal ou ses dérivés selon la revendication 1, **caractérisé en ce que** la première région variable est une région variable à chaîne légère de l'anticorps ayant la séquence en acides aminés SEQ ID No: 11; Et **en ce que** ladite deuxième région variable est une région variable de la chaîne lourde de l'anticorps ayant ladite séquence d'acides aminés dans SEQID No: 12 ou SEQ ID No: 7.

4. Un anticorps monoclonal ou ses dérivés selon l'une quelconque des revendications 1-3, comprenant une région variable de la chaîne légère de l'anticorps, une région constante de la chaîne légère de l'anticorps humain, une région charnière de la région variable de la chaîne lourde de l'anticorps et une région constante de la chaîne lourde de l'anticorps humain, une région CH1, une région CH2 et une région CH3.

5. Un anticorps monoclonal ou ses dérivés selon la revendication 4, **caractérisé en ce que** la région constante de la chaîne légère de l'anticorps humain est celle de l'anticorps humain, une chaîne kappa ou une chaîne lambda, la région constante de la chaîne lourde de l'anticorps humain étant choisie parmi les isoformes humaines IgG1, IgG2, IgG3 et IgG4.

6. Un molécule d'ADN ou gène codant pour un anticorps monoclonal ou ses dérivés selon la revendication 3, **caractérisé en ce que** la séquence nucléotidique de la région variable de la chaîne légère de l'anticorps est telle que décrite dans SEQ ID No: 1 ou SEQ ID No: 13 et la séquence nucléotidique de la région variable de la chaîne lourde de l'anticorps est telle que décrite dans SEQ ID No: 6 ou SEQ ID No: 14.

7. Un vecteur d'expression comprenant la séquence d'ADN d'une molécule d'ADN selon la revendication 6 et une séquence régulatrice de l'expression liée de manière opérationnelle à cette séquence d'ADN.

8. Une cellule hôte recombinante, **caractérisée en ce que** ladite cellule hôte recombinante est transfectée avec un vecteur d'expression selon la revendication 7.

9. Une cellule hôte recombinante ou ses cellules progénitrices selon la revendication 8, **caractérisée en ce que** la cellule hôte recombinante ou ses cellules progénitrices expriment un anticorps monoclonal ou un dérivé de celui - ci selon la revendication 1 ou la revendication 5.

10. Un composé pharmaceutique ou une composition pharmaceutique comprenant un anticorps monoclonal ou un dérivé de celui - ci selon la revendication 1 ou la revendication 5, et un véhicule pharmaceutiquement acceptable pour le traitement des tumeurs.

11. Un composé pharmaceutique ou une composition pharmaceutique comprenant un anticorps monoclonal ou un dérivé de celui - ci selon la revendication 1 ou la revendication 5, et un véhicule pharmaceutiquement acceptable selon la revendication 10, **caractérisé en ce que** la tumeur est une tumeur CD38 positive.

12. Un composé pharmaceutique ou une composition pharmaceutique comprenant un anticorps monoclonal ou un dérivé de celui - ci selon l'une quelconque des revendications 1 ou 5, et un vecteur médicinal selon la revendication 11, **caractérisé en ce que** la tumeur CD38 positive est un myélome humain ou un lymphome humain.

13. Un procédé de préparation d'un anticorps monoclonal ou d'un dérivé de celui - ci selon la revendication 1 ou la revendication 5, **caractérisé en ce qu'**il comprend les étapes suivantes:
a) Fourniture d'un vecteur d'expression comprenant une séquence moléculaire d'ADN codant pour un anticorps monoclonal ou un dérivé de celui - ci selon la revendication 1 ou la revendication 5 et une séquence régulatrice de l'expression liée de manière opérationnelle à ladite séquence moléculaire d'ADN;
b) Transfection des cellules hôtes avec le vecteur d'expression de l'étape a);
c) La culture des cellules hôtes issues de l'étape b) dans des conditions adaptées à l'expression de l'anticorps monoclonal ou de ses dérivés, et
d) L'anticorps monoclonal ou leur dérivés sont isolés, purifiés et collectés à partir du milieu de culture cellulaire hôte par chromatographie d'affinité.
